# EUROPEAN PATENT SPECIFICATION

(11) **EP 1 615 698 B1**
(45) Date of publication and mention of the grant of the patent: **29.09.2010**
(21) Application number: 04725891.8
(22) Date of filing: 06.04.2004
(51) Int. Cl.: A61P 3/04, C07D 231/14, A61K 31/16, A61K 31/415

(54) **NEW AMIDE DERIVATIVES AND PHARMACEUTICAL USE THEREOF**
NEUE AMIDE DERIVATE UND DEREN PHARMAZEUTISCHE VERWENDUNGEN
NOUVELLES AMIDES SUBSTITUES ET LEUR UTILISATION PHARMACEUTIQUE

(30) Priority: 11.04.2003 DK 200300565; 02.05.2003 US 467800 P; 27.06.2003 DK 200300972; 30.06.2003 DK 200300988; 30.06.2003 DK 200300989; 30.06.2003 DK 200300990; 02.07.2003 DK 200300998; 10.07.2003 US 486095 P; 10.07.2003 US 486097 P; 10.07.2003 US 486094 P; 10.07.2003 US 486078 P; 10.07.2003 US 486098 P; 22.12.2003 DK 200301910; 06.01.2004 DK 200400009; 16.01.2004 US 537099 P
(43) Date of publication of application: 18.01.2006
(60) Divisional application: 10164336.9
(73) Proprietor: High Point Pharmaceuticals, LLC, High Point, NC 27265 (US)
(72) Inventor: ANDERSEN, Henrik, Sune, Kgs. Lyngby, 2800 (DK); KAMPEN, Gita, Camilla, Tejlgaard, Naerum, 2850 (DK); CHRISTENSEN, Inge Thoger, Kgs. Lyngby, 2800 (DK); MOGENSEN, John, Patrick, Herlev, 2730 (DK); LARSEN, Annette Rosendal, Kgs. Lyngby, 2800 (DK); KILBURN, John Paul, Haslev, 4690 (DK)
(74) Representative: Russell, Lindsey
(86) International application number: PCT/DK2004/000250
(87) International publication number: WO 2004/089470

(56) References cited:
- EP-A- 1 176 140
- WO-A-03/000649
- WO-A-03/037274
- FR-A- 2 795 726
- US-A- 4 350 696
- US-A- 5 260 325
- MASSA, SILVIO ET AL: "Heterocyclic system. XI. Synthesis of 1H,4H-pyrazolo[4,3-b]pyrrolizine and 2H,4H-pyrazolo[4,3-b]pyrrolizine derivatives" JOURNAL OF HETEROCYCLIC CHEMISTRY , 27(6), 1805-8 CODEN: JHTCAD; ISSN: 0022-152X, 1990, XP008033087
- GIACOMELLI, GIAMPAOLO ET AL: "Microwave-assisted solution-phase synthesis of 1,4,5-trisubstituted pyrazoles" EUROPEAN JOURNAL OF ORGANIC CHEMISTRY , (3), 537-541 CODEN: EJOCFK; ISSN: 1434-193X, 2003, XP001197335
- DONOHUE, BRIDGET A. ET AL: "Design, Synthesis, and Biological Evaluation of a Library of 1-(2-Thiazolyl)-5-(trifluoromethyl)pyrazol e-4-carboxamides" JOURNAL OF COMBINATORIAL CHEMISTRY , 4(1), 23-32 CODEN: JCCHFF; ISSN: 1520-4766, 2002, XP001197336

## Description

### FIELD OF INVENTION

The present invention rotates to use of substituted amides and pharmaceutical compositions comprising the compounds according to the claims or treating disorders where it is desirable to modulate the activity of 11 β-hydroxysteroid dehydrogenase type 1 (11βHSD1).

The present invention also relates to novel substituted amides, to their use in therapy, to pharmaceutical compositions comprising the compounds, and to the use of said compounds in the manufacture of medicaments. The present compounds modulate the activity of 11β-hydroxysteroid dehydrogenase type 1 (11βHSD1) and are accordingly useful in the treatment of diseases In which such a modulation is beneficial, such as the metabolic syndrome.

### BACKGROUND OF THE INVENTION

The metabolic syndrome is a major global health problem. In the US, the prevalence in the adult population is currently estimated to be approximately 25%, and it continues to increase both in the US and worldwide. The metabolic syndrome is characterised by a combination of insulin resistance, dyslipidemia, obesity and hypertension leading to increased morbidity and mortality of cardiovascular diseases. People with the metabolic syndrome are at Increase risk of developing frank type 2 diabetes, the prevalence of which is equally escalating.

In type 2 diabetes, obesity and dysiipidemia are also highly prevalent and around 70% of people with type 2 diabetes additionally have hypertension once again leading to increased mortality of cardiovascular diseases.

In the clinical setting, it has long been known that glucocorticoids are able to induce all of the cardinal features of the metabolic syndrome and type 2 diabetes.

11β-hydroxysteroid dehydrogenase type 1 (11βHSD1) catalyses the local generation of active glucocorticoid in several tissues and organs including predominantly the liver and adipose tissue, but also e.g. skeletal muscle, bone, pancreas, endothelium, ocular tissue and certain parts of the central nervous system. Thus, 11βHSD1 serves as a local regulator of glucocorticoid actions in the tissues and organs where it is expressed (Tannin et al., J. Biol. Chem., 266, 16653 (1991); Bujalska et al., Endocrinology, 140, 3188 (1999); Whorwood et al., J Clin Endocrinol Metab., 86, 2296 (2001); Cooper et al., Bone, 27, 375 (2000); Davani et al., J. Biol. Chem., 275, 34841 (2000); Brem et al., Hypertension, 31, 459 (1998); Rauz et al., Invest. Ophthalmo/. Vis. Sci., 42, 2037 (2001); Moisan et al., Endocrinology, 127, 1450 (1990)).

The role of 11βHSD1 in the metabolic syndrome and type 2 diabetes is supported by several lines of evidence. In humans, treatment with the non-specific 11βHSD1 inhibitor carbenoxolone improves insulin sensitivity in lean healthy volunteers and people with type 2 diabetes. Likewise, 11βHSD1 knock-out mice are resistant to insulin resistance induced by obesity and stress. Additionally, the knock-out mice present with an anti-atherogenic lipid profile of decreased VLDL triglycerides and increased HDL-cholesterol. Conversely, mice that overexpress 11βHSD1 in adipocytes develop insulin resistance, hyperlipidemia and visceral obesity, a phenotype that resembles the human metabolic syndrome (Andrews et al., J. Clin. Endocrinol. Metab., 88, 285 (2003); Walker et al., J. Clin. Endocrino/. Metab., 80, 3155 (1995); Morton et al., J. Biol. Chem., 276, 41293 (2001); Kotelevtsev et al., Proc. Natl. Acad. Sci. USA, 94, 14924 (1997); Masuzaki et al., Science, 294, 2166 (2001)).

The more mechanistic aspects of 11βHSD1 modulation and thereby modulation of intracellular levels of active glucocorticoid have been investigated in several rodent models and different cellular systems. 11βHSD1 promotes the features of the metabolic syndrome by increasing hepatic expression of the rate-limiting enzymes in gluconeogenesis, namely phosphoenolpyuvate carboxykinase and glucose-6-phosphatase, promoting the differentiation of preadipocytes into adipocytes thus facilitating obesity, directly and indirectly stimulating hepatic VLDL secretion, decreasing hepatic LDL uptake and increasing vessel contractility (Kotelevtsev et al., Proc. Natl. Acad Sci. USA, 94, 14924(1997); Morton et al., J. Biol. Chem. 276, 41293 (2001); Bujalska et al., Endocrinology, 140, 3188 (1999); Souness et al., Steroids, 67, 195 (2002), Brindley & Salter, Prog. Lipid Res., 30, 349 (1991)).

WO 01/90090, WO 01/90091, WO 01/90092, WO 01/90093 and WO 01/90094 discloses various thiazol-sulfonamides as inhibitors of the human 11β-hydroxysteroid dehydrogenase type 1 enzyme, and further states that said compounds may be useful in treating diabetes, obesity, glaucoma, osteoporosis, cognitive disorders, immune disorders and depression.

We have now found substituted amides that modulate the activity of 11βHSD1 leading to altered intracellular concentrations of active glucocorticoid. More specifically, the present compounds inhibit the activity of 11βHSD1 leading to decreased intracellular concentrations of active glucocorticoid. Thus, the present compounds can be used to treat disorders where a decreased level of active intracellular glucocorticoid is desirable, such as e.g. the metabolic syndrome, type 2 diabetes, impaired glucose tolerance (IGT), impaired fasting glucose (IFG), dyslipidemia, obesity, hypertension, diabetic late complications, cardiovascular diseases, arteriosclerosis, atherosclerosis, myopathy, muscle wasting, osteoporosis, neurodegenerative and psychiatric disorders, and adverse effects of treatment or therapy with glucocorticoid receptor agonists.

One object of the present invention is to provide compounds, pharmaceutical compositions and use of compounds that modulate the activity of 11βHSD1.

### DEFINITIONS

In the following structural formulas and throughout the present specification, the following terms have the indicated meaning:

The term "halo" includes fluorine, chlorine, bromine, and iodine.

The term "trihalomethyl" includes trifluoromethyl, trichloromethyl, tribromomethyl, and triiodomethyl.

The term "trihalomethoxy" includes trifluorometoxy, trichlorometoxy, tribromometoxy, and triiodometoxy.

The term "alkyl" includes C₁-C₈ straight chain saturated and methylene aliphatic hydrocarbon groups, C₃-C₈ branched saturated hydrocarbon groups having the specified number of carbon atoms. For example, this definition shall include but is not limited to methyl (Me), ethyl (Et), propyl (Pr), butyl (Bu), pentyl, hexyl, isopropyl (i-Pr), isobutyl (i-Bu), *tert*-butyl (*t*-Bu), *sec*-butyl (s-Bu), isopentyl, neopentyl, and the like.

The term "alkenyl" includes C₂-C₆ straight chain unsaturated aliphatic hydrocarbon groups and branched C₃-C₆ unsaturated aliphatic hydrocarbon groups having the specified number of carbon atoms. For example, this definition shall include but is not limited to ethenyl, propenyl, butenyl, pentenyl, hexenyl, methylpropenyl, methylbutenyl and the like.

The term "alkynyl" includes C₂-C₆ straight chain unsaturated aliphatic hydrocarbon groups and C₄-C₆ branched unsaturated aliphatic hydrocarbon groups having the specified number of carbon atoms. For example, this definition shall include but is not limited to ethynyl, propynyl, butynyl, pentynyl, hexynyl, methylbutynyl, and the like.

The term "saturated or partially saturated cyclic, bicyclic or tricyclic ring system" represents but are not limit to aziridinyl, azepanyl, azocanyl, pyrrolinyl, pyrrolidinyl, 2-imidazolinyl, imidazolidinyl, 2-pyrazolinyl, morpholinyl, piperidinyl, thiomorpholinyl, piperazinyl, phthalimide, 1,2,3,4-tetrahydro-quinolinyl, 1,2,3,4-tetrahydro-isoquinolinyl, 1,2,3,4-tetrahydro-quinoxalinyl, indolinyl, 1, 6-aza-bicyclo[3.2.1]octane, 2-aza-bicyclo[4.1.1]octane, 2-aza-bicyclo[3.2.1]octanyl, 7-aza-bicyclo[4.1.1]octanyl, 9-aza-bicyclo[3.3.2]decanyl, 4-aza-tricyclo[4.3.1.1^{3,8}]undecanyl, 9-aza-tricyclo[3.3.2.0^{3,7}]decanyl.

The term "saturated or partially saturated cyclic ring system" represents but are not limited to cyclopropyl, cyclobutyl, cyclopentyl, cyclohexyl, cyclobutenyl, cyclopentenyl, cyclohexenyl, cycloheptenyl, cyclooctenyl, cyclononenyl, cyclodecenyl, tetrahydrofuranyl or tetrahydropyranyl.

The term "saturated or partially saturated aromatic ring system" represents but are not limited to cyclopentyl, cyclohexyl, cyclobutenyl, cyclopentenyl, cyclohexenyl, cycloheptenyl, cyclooctenyl, cyclononenyl, cyclodecenyl, tetrahydrofuranyl, tetrahydropyranyl, phenyl, pyridyl or pyrimidinyl.

The term "cycloalkyl" (e.g. cyclopropyl, cyclobutyl, cyclopentyl, cyclohexyl, cycloheptyl, cyclooctyl, cyclononyl, cyclodecyl, bicyclo[3.2.1]octyl, spiro[4.5]decyl, norpinyl, norbonyl, norcaryl, adamantyl and the like) represents a saturated, mono-, bi-, tri- or spirocarbocyclic group having the specified number of carbon atoms.

The term "cycloalkylalkyl" (e.g. cyclopropylmethyl, cyclobutylethyl, adamantylmethyl and the like) represents a cycloalkyl group as defined above attached through an alkyl group having the indicated number of carbon atoms or substituted alkyl group as defined above.

The term "cycloalkenyl" (e.g. cyclobutenyl, cyclopentenyl, cyclohexenyl, cycloheptenyl, cyclooctenyl, cyclononenyl, cyclodecenyl and the like) represents a partially saturated, mono-, bi-, tri- or spirocarbocyclic group having the specified number of carbon atoms.

The term "cycloalkylcarbonyl" (e.g. cyclopropylcarbonyl, cyclohexylcarbonyl) represents an cycloalkyl group as defined above having the indicated number of carbon atoms attached through a carbonyl group.

The term "hetcycloalkylcarbonyl" (e.g. 1-piperidin-4-yl-carbonyl, 1-(1,2,3,4-tetrahydro-isoquinolin-6-yl)carbonyl) represents an hetcycloalkyl group as defined above having the indicated number of carbon atoms attached through a carbonyl group.

The term "cycloalkylalkylcarbonyl" (e.g. cyclohexylmethylcarbonyl, cycloheptylethylcarbonyl and the like) represents a cycloalkyl group as defined above attached through an alkyl group having the indicated number of carbon atoms or substituted alkyl group as defined above.

The term "hetcycloalkyl" (tetrahydrofuranyl, tetrahydropyranyl, tertahydrothiopyranyl, piperidine, pyridzine and the like) represents a saturated mono-, bi-, tri- or spirocarbocyclic group having the specified number of carbon atoms and one or two additional heteroatoms or groups selected from nitrogen, oxygen, sulphur, SO or SO₂.

The term "hetcycloalkylalkyl" (e.g. tetrahydrofuranylmethyl, tetrahydropyranylethyl, tertahydrothiopyranylmethyl, and the like) represents a hetcycloalkyl group as defined above attached through an alkyl group having the indicated number of carbon atoms or substituted alkyl group as defined above.

The term "alkyloxy" (e.g. methoxy, ethoxy, propyloxy, allyloxy, cyclohexyloxy) represents an alkyl group as defined above having the indicated number of carbon atoms attached through an oxygen bridge.

The term "alkyloxyalkyl" (e.g. methyloxymethyl and the like) represents an alkyloxy group as defined above attached through an "alkyl" group.

The term "aryloxyhetaryl" (e.g. 2-phenoxy-pyridyl and the like) represents an aryloxy group as defined below attached through a "hetaryl" group.

The term "aryloxy" (e.g. phenoxy, naphthyloxy and the like) represents an aryl group as defined below attached through an oxygen bridge.

The term "hetaryloxy" (e.g. 2-pyridyloxy and the like) represents a hetaryl group as defined below attached through an oxygen bridge.

The term "arylalkyloxy" (e.g. phenethyloxy, naphthylmethyloxy and the like) represents an arylalkyl group as defined below attached through an oxygen bridge.

The term "hetarylalkyloxy" (e.g. 2-pyridylmethyloxy and the like) represents a hetarylalkyl group as defined below attached through an oxygen bridge.

The term "alkyloxycarbonyl" (e.g. methylformiat, ethylformiat and the like) represents an alkyloxy group as defined above attached through a carbonyl group.

The term "aryloxycarbonyl" (e.g. phenylformiat, 2-thiazolylformiat and the like) represents an aryloxy group as defined above attached through a carbonyl group.

The term "arylalkyloxycarbonyl" (e.g. benzylformiat, phenyletylformiat and the like) represents an "arylalkyloxy" group as defined above attached through a carbonyl group.

The term "alkylthio" (e.g. methylthio, ethylthio and the like) represents an alkyl group as defined above attached through a sulphur bridge.

The term "arylthio" (e.g. benzenthiol, naphthylthiol and the like) represents an aryl group as defined below attached through a sulphur bridge.

The term "hetarylthio" (e.g. pyridine-2-thiol, thiazole-2-thiol and the like) represents an hetaryl group as defined below attached through a sulphur bridge.

The term "arylthioalkyl" (e.g. methylsulfanyl benzene, ethylsulfanyl naphthalene and the like) represents an arylthio group as defined below attached through an alkyl group having the indicated number of carbon atoms.

The term "hetarylthioalkyl" (e.g. 2-methylsulfanyl-pyridine, 1-ethylsulfanyl-isoquinoline and the like) represents a hetarylthio group as defined below attached through an alkyl group having the indicated number of carbon atoms.

The term "hetaryloxyaryl" (e.g. 1-phenoxy-isoquinolyl, 2-phenoxypyridyl and the like) represents a hetaryloxy group as defined above attached through an "aryl" group as defined below.

The term "hetaryloxyhetaryl" (e.g. 1-(2-pyridyloxy-isoquinoline), 2-(imidazol-2-yloxypyridine) and the like) represents a hetaryloxy group as defined above attached through a "hetaryl" group as defined below.

The term "aryloxyalkyl" (e.g. phenoxymethyl, naphthyloxyethyl and the like) represents an aryloxy group as defined above attached through an "alkyl" group having the indicated number of carbon atoms.

The term "aryloxyaryl" (e.g. 1-phenoxy-naphthalene, phenyloxyphenyl and the like) represents an aryloxy group as defined above attached through an "aryl" group as defined below.

The term "arylalkyloxyalkyl" (e.g. ethoxymethyl-benzene, 2-methoxymethylnaphthalene and the like) represents an arylalkyloxy group as defined above attached through an "alkyl" group having the indicated number of carbon atoms.

The term "hetaryloxyalkyl" (e.g. 2-pyridyloxymethyl, 2-quinolyloxyethyl and the like) represents a hetaryloxy group as defined above attached through an "alkyl" group having the indicated number of carbon atoms.

The term "hetarylalkyloxyalkyl" (e.g. 4-methoxymethyl-pyrimidine, 2-methoxymethylquinoline and the like) represents a hetarylalkyloxy group as defined above attached through an "alkyl" group having the indicated number of carbon atoms.

The term "arylalkyl" (e.g. benzyl, phenylethyl, 3-phenylpropyl, 1-naphtylmethyl, 2-(1-naphtyl)ethyl and the like ) represents an aryl group as defined below attached through an alkyl having the indicated number of carbon atoms or substituted alkyl group as defined above.

The term "hetarylalkyl" or "hetaralkyl" (e.g. (2-furyl)methyl, (3-furyl)methyl, (2-thienyl)methyl, (3-thienyl)methyl, (2-pyridyl)methyl, 1-methyl-1-(2-pyrimidyl)ethyl and the like) represents a hetaryl group as defined below attached through an alkyl having the indicated number of carbon atoms or substituted alkyl group as defined above.

The term "alkylcarbonyl" (e.g. octylcarbonyl, pentylcarbonyl, 3-hexenylcarbonyl) represents an alkyl group as defined above having the indicated number of carbon atoms attached through a carbonyl group.

The term "arylcarbonyl" (e.g. benzoyl) represents an aryl group as defined below attached through a carbonyl group.

The term "hetarylcarbonyl" (e.g. 2-thiophenylcarbonyl, 3-methoxy-anthrylcarbonyl, oxazolylcarbonyl and the like) represents a hetaryl group as defined below attached through a carbonyl group.

The term "carbonylalkyl" (e.g. acetyl and the like) represents a carbonyl group attached through alkyl group as defined above having the indicated number of carbon atoms.

The term "alkylcarbonylalkyl" (e.g. propan-2-one, 4,4-dimethyl-pentan-2-one and the like) represents an alkylcarbonyl group as defined above attached through an alkyl group as defined above having the indicated number of carbon atoms.

The term "arylcarbonylalkyl" (e.g. 1-phenyl-propan-1-one, 1-(3-chloro-phenyl)-2-methyl-butan-1-one and the like) represents a arylcarbonyl group as defined above attached through an alkyl group as defined above having the indicated number of carbon atoms.

The term "hetarylcarbonylalkyl" (e.g. 1-pyridin-2-yl-propan-1-one, 1-(1-*H*-imidazol-2-yl)-propan-1-one and the like) represents a hetarylcarbonyl group as defined above attached through an alkyl group as defined above having the indicated number of carbon atoms.

The term "arylalkylcarbonyl" (e.g. phenylpropylcarbonyl, phenylethylcarbonyl and the like) represents an arylalkyl group as defined above having the indicated number of carbon atoms attached through a carbonyl group.

The term "hetarylalkylcarbonyl" (e.g. imidazolylpentylcarbonyl and the like) represents a hetarylalkyl group as defined above wherein the alkyl group is in turn attached through a carbonyl.

The term "alkylcarbonylamino" (e.g. methylcarbonylamino, cyclopentylcarbonylaminomethyl, methylcarbonylaminophenyl) represents an "alkylcarbonyl" group as defined above wherein the carbonyl is in turn attached through the nitrogen atom of an amino group. The nitrogen atom may itself be substituted with an alkyl or aryl group.

The term "alkylcarbonylaminoalkyl" (e.g.N-propyl-acetamide, N-butyl-propionamide and the like) represents an "alkylcarbonylamino" group attached through an alkyl group as defined above having the indicated number of carbon atoms.

The term "arylalkylcarbonylamino" (e.g. phenylacetamide, 3phenyl-propionamide and the like) represents an "arylalkylcarbonyl" group as defined above attached through an amino group.

The term "arylalkylcarbonylaminoalkyl" (e.g. N-ethyl-phenylacetamide, N-butyl-3-phenyl-propionamide and the like) represents an "arylalkylcarbonylamino" group attached through an alkyl group as defined above having the indicated number of carbon atoms.

The term "arylcarbonylamino" (e.g. benzamide, naphthalene-1-carboxylic acid amide and the like) represents an "arylcarbonyl" group as defined above attached through an amino group.

The term "arylcarbonylaminoalkyl" (e.g. N-propyl-benzamide, N-Butyl-naphthalene-1-carboxylic acid amide and the like) represents an "arylcarbonylamino" group attached through an alkyl group as defined above having the indicated number of carbon atoms.

The term "alkylcarboxy" (e.g. heptylcarboxy, cyclopropylcarboxy, 3-pentenylcarboxy) represents an alkylcarbonyl group as defined above wherein the carbonyl is in turn attached through an oxygen bridge.

The term "arylcarboxy" (e.g. benzoic acid and the like) represents an arylcarbonyl group as defined above wherein the carbonyl is in turn attached through an oxygen bridge.

The term "alkylcarboxyalkyl" (e.g. heptylcarboxymethyl, propylcarboxy *tert*-butyl, 3-pentylcarboxyethyl) represents an

The term "arylalkylcarboxy" (e.g. benzylcarboxy, phenylpropylcarboxy and the like) represents an arylalkylcarbonyl group as defined above wherein the carbonyl is in turn attached through an oxygen bridge.

The term "arylalkylcarboxyalkyl" (e.g. benzylcarboxymethyl, phenylpropylcarboxypropyl and the like) represents an arylalkylcarboxy group as defined above wherein the carboxy group is in turn attached through an alkyl group as defined above having the indicated number of carbon atoms.

The term "hetarylcarboxy" (e.g. pyridine-2-carboxylic acid and the like) represents a hetarylcarbonyl group as defined above wherein the carbonyl is in turn attached through an oxygen bridge.

The term "hetarylalkylcarboxy" (e.g. (1-*H*-imidazol-2-yl)-acetic acid, 3-pyrimidin-2-yl-propionic acid and the like) represents a hetarylalkylcarbonyl group as defined above wherein the carbonyl is in turn attached through an oxygen bridge.

The term "aryl" includes but is not limited to a carbocyclic aromatic ring system being either monocyclic, bicyclic, or polycyclic, such as phenyl, biphenyl, naphthyl, anthracenyl, phenanthrenyl, fluorenyl, indenyl, pentalenyl, azulenyl, biphenylenyl and the like. Aryl is also intended to include the partially hydrogenated derivatives of the carbocyclic aromatic systems enumerated above. Non-limiting examples of such partially hydrogenated derivatives are 1,2,3,4-tetrahydronaphthyl, 1,4-dihydronaphthyl and the like.

The term "aryl1" includes phenyl, biphenyl, naphthyl, anthracenyl, phenanthrenyl, and fluorenyl.

The term "aryl2" includes phenyl, biphenyl, and naphthyl.

The term "hetaryl" includes but is not limited to pyrrolyl (2-pyrrolyl), pyrazolyl (3-pyrazolyl), imidazolyl (1-imidazolyl, 2-imidazolyl, 4-imidazolyl, 5-imidazolyl), triazolyl (1,2,3-triazol-1-yl, 1,2,3-triazol-2-yl 1,2,3-triazol-4-yl, 1,2,4-triazol-3-yl), oxazolyl (2-oxazolyl, 4-oxazolyl, 5-oxazolyl), isoxazolyl (3-isoxazolyl, 4-isoxazolyl, 5-isoxazolyl), thiazolyl (2-thiazolyl, 4-thiazolyl, 5-thiazolyl), thiophenyl (2-thiophenyl, 3-thiophenyl, 4-thiophenyl, 5-thiophenyl), furanyl (2-furanyl, 3-furanyl, 4-furanyl, 5-furanyl), pyridyl (2-pyridyl, 3-pyridyl, 4-pyridyl, 5-pyridyl), 5-tetrazolyl, pyrimidinyl (2-pyrimidinyl, 4-pyrimidinyl, 5-pyrimidinyl, 6-pyrimidinyl), pyrazinyl, pyridazinyl (3-pyridazinyl, 4-pyridazinyl, 5-pyridazinyl), quinolyl (2-quinolyl, 3-quinolyl, 4-quinolyl, 5-quinolyl, 6-quinolyl, 7-quinolyl, 8-quinolyl), isoquinolyl (1-isoquinolyl, 3-isoquinolyl, 4-isoquinolyl, 5-isoquinolyl, 6-isoquinolyl, 7-isoquinolyl, 8-isoquinolyl), benzo[b]furanyl (2-benzo[b]furanyl, 3-benzo[b]furanyl, 4-benzo[b]furanyl, 5-benzo[b]furanyl, 6-benzo[b]furanyl, 7-benzo[b]furanyl), 2,3-dihydro-benzo[b]furanyl (2-(2,3-dihydro-benzo[b]furanyl), 3-(2,3-dihydro-benzo[b]furanyl), 4-(2,3-dihydro-benzo[b]furanyl), 5-(2,3-dihydrobenzo-[b]furanyl), 6-(2,3-dihydro-benzo-[b]furanyl), 7-(2,3-dihydro-benzo[b]furanyl)), benzo[b]thiophenyl (2-benzo[b]thiophenyl, 3-benzo[b]thiophenyl, 4-benzo[b]thiophenyl, 5-benzo[b]thiophenyl, 6-benzo[b]thiophenyl, 7-benzo[b]thiophenyl), 2,3-dihydro-benzo[b]thiophenyl (2-(2,3-dihydro-benzo[b]thiophenyl), 3-(2,3-dihydro-benzo[b]thiophenyl), 4-(2,3-dihydrobenzo[b]thiophenyl), 5-(2,3-dihydro-benzo[b]thiophenyl), 6-(2,3-dihydro-benzo[b]thiophenyl), 7-(2,3-dihydro-benzo[b]thiophenyl)), 4,5,6,7-tetrahydro-benzo[b]thiophenyl (2-(4,5,6,7-tetrahydro-benzo[b]thiophenyl), 3-(4,5,6,7-tetrahydro-benzo[b]thiophenyl), 4-(4,5,6,7-tetrahydro-benzo[b]thiophenyl), 5-(4,5,6,7-tetrahydro-benzo[b]thiophenyl), 6-(4,5,6,7-tetrahydro-benzo[b]thiophenyl), 7-(4,5,6,7-tetrahydro-benzo[b]thiophenyl)), thieno[2,3-b]thiophenyl, 4,5,6,7-tetrahydro-thieno[2,3-c]pyridyl (4-(4,5,6,7-tetrahydro-thieno[2,3-c]pyridyl), 5-4,5,6,7-tetrahydro-thieno[2,3-c]pyridyl), 6-(4,5,6,7-tetrahydro-thieno[2,3-c]pyridyl), 7-(4,5,6,7-tetrahydro-thieno[2,3-c]pyridyl)), indolyl (1-indolyl, 2-indolyl, 3-ihdolyl, 4-indolyl, 5-indolyl, 6-indolyl, 7-indolyl), isoindolyl (1-isoindolyl, 2-isoindolyl, 3-isoindolyl, 4-isoindolyl, 5-isoindolyl, 6-isoindolyl, 7-isoindolyl), 1,3-dihydro-isoindolyl (1-(1,3-dihydro-isoindolyl), 2-(1,3-dihydroisoindolyl), 3-(1,3-dihydro-isoindolyl), 4-(1,3-dihydro-isoindolyl), 5-(1,3-dihydro-isoindolyl), 6-(1,3-dihydro-isoindolyl), 7-(1,3-dihydro-isoindolyl)), indazole (1-indazolyl, 3-indazolyl, 4-indazolyl, 5-indazolyl, 6-indazolyl, 7-indazolyl), benzimidazolyl (1-benzimidazolyl, 2-benzimidazolyl, 4-benzimidazolyl, 5-benzimidazolyl, 6-benzimidazolyl, 7-benzimidazolyl, 8-benzimidazolyl), benzoxazolyl (1-benz-oxazolyl, 2-benzoxazolyl), benzothiazolyl (1-benzothiazolyl, 2-benzothiazolyl, 4-benzothiazolyl, 5-benzothiazolyl, 6-benzothiazolyl, 7-benzothiazolyl), benzo-[1,2,5]oxadiazolyl, (4-benzo[1,2,5]oxadiazole, 5-benzo[1,2,5]oxadiazole), carbazolyl (1-carbazolyl, 2-carbazolyl, 3-carbazolyl, 4-carbazolyl), piperidinyl (2-piperidinyl, 3-piperidinyl, 4-piperidinyl), pyrrolidinyl (1-pyrrolidinyl, 2-pyrrolidinyl, 3-pyrrolidinyl).

The term "alkylSOₘ" (e.g. ethylsulfonyl, ethylsulfinyl and the like) represents an alkyl group as defined above, wherein the alkyl group is in turn attached through a sulphur bridge wherein the sulphur is substituted with m oxygen atoms.

The term "arylSOₘ" (e.g. phenylsulfinyl, naphthyl-2-sulfonyl and the like) represents an aryl group as defined above, wherein the aryl group is in turn attached through a sulphur bridge wherein the sulphur is substituted with m oxygen atoms.

The term "hetarylSOₘ" (e.g. thiazol-2-sulfinyl, pyridine-2-sulfonyl and the like) represents a hetaryl group as defined above, wherein the hetaryl group is in turn attached through a sulphur bridge wherein the sulphur is substituted with m oxygen atoms.

With respect to formula III, the term "alkylNR⁵alkyl" (e.g. N-ethyl-N-isobutyl-amine, N,N-dimethylamine and the like wherein the amino group (N) is substituted with R⁵ as defined below) represents an alkylNR⁵ group as defined above attached through an "alkyl" group.

With respect to formula III, the term "arylalkylNR⁵alkyl" (e.g. N-benzyl-N-methylamine, N-phenethyl-N-ethyhamine and the like wherein the amino group (N) is substituted with R⁵ as defined below) represents an arylalkylNR⁵ group as defined above attached through an "alkyl" group.

Certain of the above defined terms may occur more than once in the structural formulae, and upon such occurrence each term shall be defined independently of the other.

The term "optionally substituted" as used herein means that the groups in question are either unsubstituted or substituted with one or more of the substituents specified. When the groups in question are substituted with more than one substituent, the substituents may be the same or different.

The term "treatment" is defined as the management and care of a patient for the purpose of combating or alleviating the disease, condition or disorder, and the term includes the administration of the active compound to prevent the onset of the symptoms or complications, or alleviating the symptoms or complications, or eliminating the disease, condition, or disorder.

The term "pharmaceutically acceptable" is defined as being suitable for administration to humans without adverse events.

### DETAILED DESCRIPTION OF THE INVENTION

In one aspect, the present disclosure provides the use of a substituted amide according to the claim 5, a prodrug thereof, or a salt thereof with a pharmaceutically acceptable acid or base, or any optical isomer or mixture of optical isomers, including a racemic mixture, or any tautomeric forms for
a) modulation of the activity of 11βHSD1 ;or
b) inhibition of 11βHSD1,
   in a patient in need thereof.

In another aspect, the present disclosure provides the use of a substituted amide according to the claims, or a salt thereof with a pharmaceutically acceptable acid or base, or any optical isomer or mixture of optical isomers, including a racemic mixture, or any tautomeric forms for the preparation of a pharmaceutical composition for the treatment, prevention and/or prophylaxis of any disorder and disease where it is desirable to
a) modulate the activity of 11βHSD1; or
b) inhibit 11βPHSD1,
   in a patient in need thereof.

The present invention is concerned the substituted amides of the general formula (III) 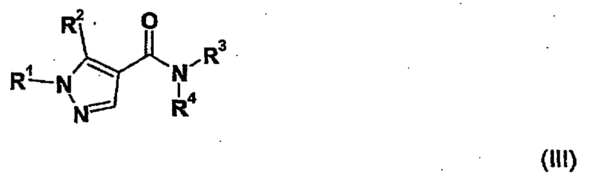 wherein
R¹ is aryl, arylC₁-C₈alkyl, hetaryl or hetaryfC₁-C₆alkyl optionally substituted with one or more of R⁶ independently;
R² is halo, C₁-C₆alkyl, C₂-C₆alkenyl, C₂-C₆alkynyl, C₃-C₁₀cycloalkyl, C₃-C₁₀cycloalkylC₁-C₈-alkyl, trihalomethyl, aryl, arylC₁-C₆alkyl, C₁-C₆alkyloxyC₁-C₆alkyl, arylC₁-C₆alkyloxyC₁-C₆alkyl, C₁-C₆alkylNR⁵C₁-C₆alkyl, arylC₁-C₆alkylNR⁶C₁-C₆alkyl, hetaryl or hetarylC₁-C₆alkyl wherein the alkyl, alkenyl, alkynyl, cycloalkyl and aryl groups independently are optionally substituted with one or more R⁷;
R³ is C₁-C₆alkyl optionally substituted with one or more of R⁸;
R⁴ is C₆-C₁₀cycloalkyl, C₆-C₁₀hetcycloalkyl, C₆-C₁₀cycloalkylC₁-C₆alkyl or C₆-C₁₀hetcycloalkylC₁-C₆alkyl wherein the alkyl, cycloalkyl and hetcycloalkyl groups independently are optionally substituted with one or more of R⁸; or
R³ and R⁴ together with the nitrogen to which they are attached, are forming a saturated or partially saturated bicyclic/bridge ring system containing from 7 to 12 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, hetaryl-C₁-C₆alkyl, hydroxy, oxo, C₁-C₆alkyloxy, arylC₁-C₆alkyloxy or hetarylC₁-C₆alkyloxy, wherein the alkyl and aryl groups independently are optionally substituted with one ore more of R⁹;
R⁵ is C₁-C₆alkyl, C₂-C₆alkenyl, C₂-C₆alkynyl, C₃-C₁₀cycloalkyl, C₃-C₁₀hetcycloalkyl, C₃-C₁₀-cycloalkylC₁-C₆alkyl, C₃-C₁₀hetcycloalkylC₁-C₆alkyl, aryl, hetaryl, arylC₁-C₆alkyl or hetarylC₁-C₆alkyl wherein the alkyl, alkenyl, alkynyl, aryl, hetaryl, cycloalkyl and hetcycloalkyl groups independently are optionally substituted with one or more of R⁹;
R⁶ and R⁷ independently are hydrogen, hydroxy, oxo, halo, nitro, cyano, C₁-C₆alkyl, C₁-C₆-alkyloxy, trihalomethyl, trihalomethoxy, NR¹⁰R¹¹, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, C₁-C₆alkyloxycarbonyl, aryloxycarbonyl, arylC₁-C₆alkyloxycarbonyl, C₁-C₆alkylcarboxy, arylcarboxy or arylC₁-C₆alkylcarboxy;
R⁸ and R⁹ independently are hydrogen, C₁-C₆alkyl, aryl, hetaryl, arylC₁-C₆alkyl, hetarylC₁-C₆-alkyl, hydroxy, oxo, cyano, NR¹⁰R¹¹, C₁-C₆alkyloxy, aryloxy, arylC₁-C₆alkyloxy_{;} hetaryloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy;
R¹⁰ and R¹¹ independently are hydrogen, C₁-C₈alkyl, aryl, hetaryl, arylC₁-C₆alkyl, C₃-C₁₀-cycloalkyl, C₃-C₁₀hetcycloalkyl, C₃-C₁₀cycloalkylC₁-C₆alkyl, C₁-C₆alkylcarbonyl, C₁-C₆alkylcarboxyC₁-C₆alkyl; or
R¹⁰ and R¹¹ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-₆-alkylcarboxy; or
a salt thereof with a pharmaceutically acceptable acid or base, or any optical isomer or mixture of optical isomers, including a racemic mixture, or any tautomeric forms.

In another embodiment, the present invention is concerned with compounds or prodrugs thereof of the general formula (III) wherein:
R¹ is aryl or hetaryl optionally substituted with one or more R⁶ independently;
R² is halo, C₁-C₆alkyl, C₂-C₆alkenyl, C₂-C₆alkynyl, C₃-C₁₀cycloalkyl, C₃-C₁₀cycloalkylC₁-C₆-alkyl, aryl, arylC₁-C₆alkyl, C₁-C₆alkyloxyC₁-C₆alkyl, arylC₁-C₆alkyloxyC₁-C₆alkyl, C₁-C₆-alkylNR⁵C₁-C₆alkyl, arylC₁-C₆alkylNR⁵C₁-C₆alkyl, hetaryl or hetarylC₁-C₆alkyl wherein the alkyl, alkenyl, alkynyl, cycloalkyl and aryl groups independently are optionally substituted with one or more R⁷;
R³ is C₁-C₆alkyl optionally substituted with one or more of R⁸;
R⁴ is C₆-C₁₀cycloalkyl, C₆-C₁₀hetcycloalkyl, C₆-C₁₀cycloalkylC₁-C₆alkyl or C₆-C₁₀hetcycloalkylC₁-C₆alkyl wherein the alkyl, cycloalkyl and hetcycloalkyl groups independently are optionally substituted with one or more of R⁸;
R⁵ is C₁-C₆alkyl, C₂-C₆alkenyl, C₂-C₆alkynyl, C₃-C₁₀cycloalkyl, C₃-C₁₀hetcycloalkyl, C₃-C₁₀-cycloalkylC₁-C₆alkyl, C₃-C₁₀hetcycloalkylC₁-C₆alkyl, aryl, hetaryl, arylC₁-C₆alkyl or hetarylC₁-C₆alkyl wherein the alkyl, alkenyl, alkynyl, aryl, hetaryl, cycloalkyl and hetcycloalkyl groups independently are optionally substituted with one or more of R⁹;
R⁶ and R⁷ independently are hydrogen, hydroxy, oxo, halo, nitro, cyano, C₁-C₆alkyl, C₁-C₆-alkyloxy, trihalomethyl, trihalomethoxy, NR¹⁰R¹¹, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, C₁-C₆alkyloxycarbonyl, aryloxycarbonyl, arylC₁-C₆alkyloxycarbonyl, C₁-C₆alkylcarboxy, arylcarboxy or arylC₁-C₆alkylcarboxy;
R⁸ and R⁹ independently are hydrogen, G₁-C₆alkyl, aryl, hetaryl, arylC₁-C₆alkyl, hetarylC₁-C₆-alkyl, hydroxy, oxo, cyano, NR¹⁰R¹¹, C₁-C₆alkyloxy, aryloxy, arylC₁-C₆alkyloxy, hetaryloxy, hetarylC₁-C₆alkyloxy, C₁-C₆alkyloxyC₁-C₆alkyl C₁-C₆alkyl-carbonyl, arylcarbonyl, hetarylcarbonyl, arylC₁-C₆alkylcarbonyl, hetarylC₁-C₆alkyl-carbonyl, C₁-C₆alkylcarboxy, arylcarboxy or arylC₁-C₆alkylcarboxy;
R¹⁰ and R¹¹ independently are hydrogen, C₁-C₈alkyl, aryl, hetaryl, arylC₁-C₈alkyl; C₃-C₁₀-cycloalkyl, C₃-C₁₀hetcycloalkyl, C₃-C₁₀cycloalkylC₁-C₈alkyl, C₁-C₆alkylcarboxyC₁-C₆alky); or
R¹⁰ and R¹¹ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetaryl C₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylarbonyl, C₁-C₆alkylcarboxy, arylcarboxy or arylC₁-₆-alkylcarboxy; or
a salt thereof with a pharmaceutically acceptable acid or base, or any optical isomer or mixture of optical isomers, including a racemic mixture, or any tautomeric forms.

In another embodiment of the present invention, in formula (III) R¹ is aryl, arylC₁-C₆alkyl or hetaryl optionally substituted with one or more of R⁶.

In another embodiment of the present invention, in formula (III) R¹ is aryl optionally substituted with one or more of R⁶.

In another embodiment of the present invention, in formula (III) R¹ is arylC₁-C₆alkyl optionally substituted with one or more of R⁶.

In another embodiment of the present invention, in formula (III) R¹ is hetaryl optionally substituted with one or more of R⁶.

In another embodiment of the present invention, in formula (III) R² is C₁-C₆alkyl, C₃-C₁₀Cycloalkyl, C₃-C₁₀cycloalkylC₁-C₆alkyl, trihalomethyl, arylC₁-C₆alkyl, or hetarylC₁-C₆alkyl wherein the alkyl, cycloalkyl and aryl groups independently are optionally substituted with one or more R⁷.

In another embodiment of the present invention, in formula (III) R² is C₁-C₆alkyl optionally substituted with one or more R⁷.

In another embodiment of the present invention, in formula (III) R² is trihalomethyl.

In another embodiment of the present invention, in formula (III) R³ is C₁-C₆alkyl optionally substituted with one or more of R⁸.

In another embodiment of the present invention, in formula (III) R⁴ is C₈-C₁₀cycloalkyl, or C₆-C₁₀hetcycloalkyl, wherein the cycloalkyl and hetcycloalkyl groups independently are optionally substituted with one or more of R⁸.

In another embodiment of the present invention, in formula (III) R⁴ is C₆-C₁₀cycloalkyl optionally substituted with one or more of R⁸.

In another embodiment of the present Invention, in formula (III) R⁴ is C₆ C₆-C₁₀hetcycloalkyl optionally substituted with one or more of R⁸.

In another embodiment of the invention, in formula (III) R³ and R⁴ together with the nitrogen to which they are attached, are forming a saturated or partially saturated bicyclic/bridge ring system containing from 7 to 12 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, hetarylC₁-C₆alkyl, hydroxy, oxo, C₁-C₆alkyloxy, arylC₁-C₆alkyloxy or hetarylC₁-C₆alkyloxy, wherein the alkyl and aryl groups independently are optionally substituted with one ore more of R⁹.

In another embodiment of the present invention, in formula (III) the saturated or partially saturated bicyclic/bridge ring system is 6-aza-bicyclo[3.2.1]octane.

In another embodiment of the present Invention, in formula (III) R⁸ and R⁷ independently are hydrogen, hydroxy, oxo, halo, cyano, C₁-C₆alkyl, C₁-C₆alkyloxy, trihalomethyl, NR¹⁰R¹¹, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, C₁-C₆alkyloxycarbonyl, aryloxycarbonyl or arylC₁-C₆alkyloxycarbonyl.

In another embodiment of the present invention, in formula (III) R⁸ and R⁹ independently are hydrogen, C₁-C₆alkyl, hydroxy, oxo, C₁-C₆alkyloxy or arylC₁-C₆alkyloxy.

In another embodiment of the present invention, in formula (III) R¹⁰ and R¹¹ independently are hydrogen or C₁-C₈arkyl.

In another embodiment of the present invention the compound of the general formula (III) or a prodrug thereof is 1-(4-Chloro-phenyl)-5-propyl-1H-pyrazole-4-carboxylic acid cyclohexyl-methyl-amide or a salt thereof with a pharmaceutically acceptable acid or base, or any optical Isomer or mixture of optical isomers, including a racemic mixture, or any tautomeric forms.

In another embodiment of the present invention the compounds of the general formula (III) is selected from the group consisting of:
1-(4-Chloro-phenyl)-5-trifluoromethyl-1H-pyrazole-4-carboxylic acid cyclohexyl-methyl-amide;
[1-(4-Methoxy-phenyl)-5-methyl-1*H*-pyrazol-4-yl]-(1,3,3-trimethyl-6-aza-bicydo[3.2.1]oct-6-yl)-methanone; 1 [1-(4-Chloro-phenyl)-5-propyl-1*H*-pyrazol-4-yl]-(1,3,3-trimethyl-6-aza-bicyclo[3.2,1]oct-6-yl)-methanone;
[1-(3,5-Dichloro-phenyl)-5-propyl-1*H*-pyrazol-4-yl]-(1,3,3-trimethyl-6-aza-bicyclo[3.2.1]oct-6-yl)-methanone; or
a salt thereof with a pharmaceutically acceptable acid or base, or any optical isomer or mixture of optical isomers, including a racemic mixture, or any tautomeric forms.

In yet another embodiment of the present invention the compounds of the general formula (III) is selected from the group consisting of: 1-(Phenyl)-5-methyl-1*H*-pyrazole-4-carboxylic acid cyclohexyl-methyl-amide; 1-(4-Fluoro-phenyl)-5-methyl-1*H*-pyrazole-4-carboxylic acid cyclohexyl-methyl-amide; 1-(4-Methoxy-phenyl)-5-methyl-1*H*-pyrazole-4-carboxylic acid cyclohexyl-methyl-amide; 1-(4-Chloro-phenyl)-5-methyl-1*H*-pyrazole4-carboxylic acid cyclohexyl-methyl-amide; 1-(2-Methyl-phenyl)-5-methyl-1*H*-pyrazole-4-carboxylic acid cyclohexyl-methyl-amide; 1-(4-Amino-phenyl)-5-methyl-1*H*-pyrazole-4-carboxylic acid cyclohexyl-methyl-amide; 1-(2-Pyridyl)-5-methyl-1*H*-pyrazole-4-carboxylic acid cyclohexyl-methyl-amide; 1-(2-Pyrldyl)-5-propyl-1*H*-pyrazole-4-carboxylic acid cyclohexyl-methyl-amide; or a salt thereof with a pharmaceutically acceptable acid or base, or any optical isomer or mixture of optical isomers, including a racemic mixture; or any tautomeric forms.

The compounds of the present invention have asymmetric centers and may occur as racemates, racemic mixtures, and as individual enantiomers or diastereoisomers, with all isomeric forms being included in the present invention as well as mixtures thereof.

The present invention also encompasses pharmaceutically acceptable salts of the present compounds. Such salts include pharmaceutically acceptable acid addition salts, pharmaceutically acceptable base addition salts, pharmaceutically acceptable metal salts, ammonium and alkylated ammonium salts. Acid addition salts include salts of inorganic acids as well as organic acids. Representative examples of suitable inorganic acids Include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methanesulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic; p-toluenesulfonic acids, sulphates, nitrates, phosphates, perchlorates, borates, acetates, benzoates, hydroxynaphthoates, glycerophosphates, ketoglutarates and the like. Further examples of pharmaceutically acceptable inorganic or organic acid addition salts include the pharmaceutically acceptable salts listed in J. Pharm. Sci., 66, 2 (1977). Examples of metal salts include lithium, sodium, potassium, barium, calcium, magnesium, zinc, calcium salts and the like. Examples of amines and organic amines include ammonium, methylamine, dimethylamine, trimethylamine, ethylamine, diethyiamine, propylamine, butylamine, tetramethylamine, ethanolamine, diethanolamine, triethanolamine, meglumine, ethylenediamine, choline, N,N'-dibenzylethylenediamine, N-benzylphenylethylamine, N-methyl-D-giucamine, guanidine. Examples of cationic amino acids include lysine, arginine, histidine.

Further, some of the compounds of the present invention may form solvates with water or common organic solvents. Such solvates are encompassed within the scope of the invention.

The pharmaceutically acceptable salts are prepared by reacting a compound of the present invention with 1 to 4 equivalents of a base such as sodium hydroxide, sodium methoxide, sodium hydride, potassium *tert*-butoxide, calcium hydroxide, magnesium hydroxide and the like, in solvents like ether, THF, methanol, *tert*-butanol, dioxane, isopropanol, ethanol etc. Mixtures of solvents may be used. Organic bases like lysine, arginine, diethanolamine, choline, guandine and their derivatives etc. may also be used. Alternatively, acid addition salts wherever applicable are prepared by treatment with acids such as hydrochloric acid, hydrobromic acid, nitric acid, sulfuric acid, phosphoric acid, p-toluenesulphonic acid, methanesulfonic acid, acetic acid, citric acid, maleic acid salicylic acid, hydroxynaphthoic acid, ascorbic acid, palmitic acid, succinic acid, benzoic acid, benzenesulfonic acid, tartaric acid and the like in solvents like ethyl acetate, ether, alcohols, acetone, THF, dioxane etc. Mixture of solvents may also be used.

The stereoisomers of the compounds forming part of this invention may be prepared by using reactants in their single enantiomeric form in the process wherever possible or by conducting the reaction in the presence of reagents or catalysts in their single enantiomer form or by resolving the mixture of stereoisomers by conventional methods. Some of the preferred methods include use of microbial resolution, enzymatic resolution, resolving the diastereomeric salts formed with chiral acids such as mandelic acid, camphorsulfonic acid, tartaric acid, lactic acid, and the like wherever applicable or chiral bases such as brucine, (*R*)- or (*S*)-phenylethylamine, cinchona alkaloids and their derivatives and the like. Commonly used methods are compiled by Jaques et al. in "Enantiomers, Racemates and Resolution" (Wiley Interscience, 1981). More specifically the compound of the present invention may be converted to a 1:1 mixture of diastereomeric amides by treating with chiral amines, aminoacids, aminoalcohols derived from aminoacids; conventional reaction conditions may be employed to convert acid into an amide; the diastereomers may be separated either by fractional crystallization or chromatography and the stereoisomers of compound of formula I may be prepared by hydrolysing the pure diastereomeric amide.

Various polymorphs of the compounds forming part of this invention may be prepared by crystallization of said compounds under different conditions. For example, using different solvents commonly used or their mixtures for recrystallization; crystallizations at different temperatures; various modes of cooling, ranging from very fast to very slow cooling during crystallizations. Polymorphs may also be obtained by heating or melting the compound followed by gradual or fast cooling. The presence of polymorphs may be determined by solid probe nmr spectroscopy, ir spectroscopy, differential scanning calorimetry, powder X-ray diffraction or such other techniques.

It is a well known problem In drug discovery that compounds, such as enzyme inhibitors, may be very potent and selective in biochemical assays, yet be inactive *in vivo*. This lack of so-called bioavailability may be ascribed to a number of different factors such as lack of or poor absorption in the gut, first pass metabolism in the liver and/or poor uptake in cells. Although the factors determining bioavailability are not completely understood, there are many examples in the scientific literature - well known to those skilled in the art - of how to modify compounds, which are potent and selective in biochemical assays but show low or no activity *in vivo,* into drugs that are biologically active.

The compounds of the present invention, termed the 'original compound', can be modified by attaching chemical groups that will improve the bioavailability of said compounds in such a way that the uptake in cells or mammals is facilitated.

Examples of said modifications, which are not intended in any way to limit the scope of the invention, include changing of one or more carboxy groups to esters (for instance methyl esters, methyl esters, *tert*-butyl, acetoxymethyl, pivaloyloxymethyl esters or other acyloxymethyl esters). Compounds of the invention, original compounds, such modified by attaching chemical groups are termed 'modified compounds'.

The invention also encompasses active metabolites of the present compounds. The compounds according to the invention alter, and more specifically, reduce the level of active intracellular glucocorticoid and are accordingly useful for the treatment, prevention and/or prophylaxis of disorders and diseases in which such a modulation or reduction is beneficial.

Accordingly, the present compounds may be applicable for the treatment, prevention and/or prophylaxis of the metabolic syndrome, insulin resistance, dyslipidemia, hypertension, obesity, type 2 diabetes, impaired glucose tolerance (IGT), impaired fasting glucose (IFG), Latent Autoimmune Diabetes in the Adult (LADA), type 1 diabetes, diabetic late complications including cardiovascular diseases, cardiovascular disorders, disorders of lipid metabolism, neurodegenerative and psychiatric disorders, dysregulation of intraocular pressure including glaucoma, immune disorders, inappropriate immune responses, musculo-skeletal disorders, gastrointestinal disorders, polycystic ovarie syndrome (PCOS), reduced hair growth or other diseases, disorders or conditions that are influenced by intracellular glucocorticoid levels, adverse effects of increased blood levels of active endogenous or exogenous glucocorticoid, and any combination thereof, adverse effects of increased plasma levels of endogenous active glucocorticoid, Cushing's disease, Cushing's syndrome, adverse effects of glucocorticoid receptor agonist treatment of autoimmune diseases, adverse effects of glucocorticoid receptor agonist treatment of inflammatory diseases, adverse effects of glucocorticoid receptor agonist treatment of diseases with an inflammatory component, adverse effects of glucocorticoid receptor agonist treatment as a part of cancer chemotherapy, adverse effects of glucocorticoid receptor agonist treatment for surgical/post-surgical or other trauma, adverse effects of glucocorticoid receptor agonist therapy in the context of organ or tissue transplantation or adverse effects of glucocorticoid receptor agonist treatment in other diseases, disorders or conditions where glucocorticoid receptor agonists provide clinically beneficial effects.

More specifically the present compounds may be applicable for the treatment, prevention and/or prophylaxis of the metabolic syndrome, type 2 diabetes, diabetes as a consequence of obesity, insulin resistance, hyperglycemia, prandial hyperglycemia, hyperinsulinemia, inappropriately low insulin secretion, impaired glucose tolerance (IGT), impaired fasting glucose (IFG), increased hepatic glucose production, type 1 diabetes, LADA, pediatric diabetes, dyslipidemia, diabetic dyslipidemia, hyperlipidemia, hypertriglyceridemia, hyperlipoproteinemia, hypercholesterolemia, decreased HDL cholesterol, impaired LDUHDL ratio, other disorders of lipid metabolism, obesity, visceral obesity, obesity as a consequence of diabetes, increased food intake, hypertension, diabetic late complications, micro-/macroalbuminuria, nephropathy, retinopathy, neuropathy, diabetic ulcers, cardiovascular diseases, arteriosclerosis, atherosclerosis, coronary artery disease, cardiac hypertrophy, myocardial ischemia, heart insufficiency, congestional heart failure, stroke, myocardial infarction, arrythmia, decreased blood flow, erectile dysfunction (male or female), myopathy, loss of muscle tissue, muscle wasting, muscle catabolism, osteoporosis, decreased linear growth, neurodegenerative and psychiatric disorders, Alzheimers disease, neuronal death, impaired cognitive function, depression, anxiety, eating disorders, appetite regulation, migraine, epilepsia, addiction to chemical substances, disorders of intraocular pressure, glaucoma, polycystic ovary syndrome (PCOS), inappropriate immune responses, inappropriate T helper-1/T helper-2 polarisation, bacterial infections, mycobacterial infections, fungal infections, viral infections, parasitic infestations, suboptimal responses to immunizations, immune dysfunction, partial or complete baldness, or other diseases, disorders or conditions that are influenced by intracellular glucocorticoid levels and any combination thereof, adverse effects of glucocorticoid receptor agonist treatment of allergic-inflammatory diseases such as asthma and atopic dermatitis, adverse effects of glucocorticoid receptor agonist treatment of disorders of the respiratory system e.g. asthma, cystic fibrosis, emphysema, bronchitis, hypersensitivity, pneumonitis, eosinophilic pneumonias, pulmonary fibrosis, adverse effects of glucocorticoid receptor agonist treatment of inflammatory bowel disease such as Crohn's disease and ulcerative colitis; adverse effects of glucocorticoid receptor agonist treatment of disorders of the immune system, connective tissue and joints e.g. reactive arthritis, rheumatoid arthritis, Sj6gren's syndrome, systemic lupus erythematosus, lupus nephritis, Henoch-Schönlein purpura, Wegener's granulomatosis, temporal arteritis, systemic sclerosis, vasculitis, sarcoidosis, dermatomyositis-polymyositis, pemphigus vulgaris; adverse effects of glucocorticoid receptor agonist treatment of endocrinological diseases such as hyperthyroidism, hypoaldosteronism, hypopituitarism; adverse effects of glucocorticoid receptor agonist treatment of hematological diseases e.g. hemolytic anemia, thrombocytopenia, paroxysmal nocturnal hemoglobinuria; adverse effects of glucocorticoid receptor agonist treatment of cancer such as spinal cord diseases, neoplastic compression of the spinal cord, brain tumours, acute lymphoblastic leukemia, Hodgkin's disease, chemotherapy-induced nausea, adverse effects of glucocorticoid receptor agonist treatment of diseases of muscle and at the neuro-muscular joint e.g. myasthenia gravis and heriditary myopathies (e.g. Duchenne muscular dystrophy), adverse effects of glucocorticoid receptor agonist treatment in the context of surgery & transplantation e.g. trauma, post-surgical stress, surgical stress, renal transplantation, liver transplantation, lung transplantation, pancreatic islet transplantation, blood stem cell transplantation, bone marrow transplantation, heart transplantation, adrenal gland transplantation, tracheal transplantation, intestinal transplantation, corneal transplantation, skin grafting, keratoplasty, lens implantation and other procedures where immunosuppression with glucocorticoid receptor agonists is beneficial; adverse effects of glucocorticoid receptor agonist treatment of brain absess, nausea/vomiting, infections, hypercalcemia, adrenal hyperplasia, autoimmune hepatitis, spinal cord diseases, saccular aneurysms or adverse effects to glucocorticoid receptor agonist treatment in other diseases, disorders and conditions where glucocorticoid receptor agonists provide clinically beneficial effects.

Accordingly, in a further aspect the invention relates to a compound according to the invention for use as a pharmaceutical composition.

The invention also relates to pharmaceutical compositions comprising, as an active ingredient, at least one compound according to the invention together with one or more pharmaceutically acceptable carriers or diluents.

The pharmaceutical composition is preferably in unit dosage form, comprising from about 0.05 mg/day to about 2000 mg/day, preferably from about 1 mg/day to about 500 mg/day of a compound according to the invention.

In another embodiment, the patient is treated with a compound according to the invention for at least about 1 week, for at least about 2 weeks, for at least about 4 weeks, for at least about 2 months or for at least about 4 months.

In yet another embodiment, the pharmaceutical composition is for oral, nasal, transdermal, pulmonal or parenteral administration.

Furthermore, the invention relates to the use of a compound according to the invention for the preparation of a pharmaceutical composition for the treatment, prevention and/or prophylaxis of disorders and diseases wherein a modulation or an inhibition of the activity of 11βHSD1 is beneficial.

In a preferred embodiment of the invention the present compounds are used for the preparation of a medicament for the treatment, prevention and/or prophylaxis of any diseases and conditions that are influenced by intracellular glucocorticoid levels as mentioned above.

Thus, in a preferred embodiment of the invention the present compounds are used for the preparation of a medicament for the treatment, prevention and/or prophylaxis of conditions and disorders were a decreased level of active intracellular glucocorticoid is desire able, such as the conditions and diseases mentioned above.

In yet a preferred embodiment of the invention the present compounds are used for the preparation of a medicament for the treatment, prevention and/or prophylaxis of the metabolic syndrome including insulin resistance, dyslipidemia, hypertension and obesity.

In yet another preferred embodiment of the invention the present compounds are used for the preparation of a medicament for the treatment, prevention and/or prophylaxis of type 2 diabetes, impaired glucose tolerance (IGT), impaired fasting glucose (IFG).

In yet another preferred embodiment of the invention the present compounds are used for the preparation of a pharmaceutical composition for the delaying or prevention of the progression from IGT to type 2 diabetes.

In yet another preferred embodiment of the invention the present compounds are used for the preparation of a pharmaceutical composition for the delaying or prevention of the progression of the metabolic syndrome into type 2 diabetes.

In still another preferred embodiment of the invention the present compounds are used for the preparation of a pharmaceutical composition for the treatment, prevention and/or prophylaxis of diabetic late complications including cardiovascular diseases; arteriosclerosis; atherosclerosis.

In a further preferred embodiment of the invention the present compounds are used for the preparation of a pharmaceutical composition for the treatment, prevention and/or prophylaxis of neurodegenerative and psychiatric disorders.

In yet a further preferred embodiment of the invention the present compounds are used for the preparation of a pharmaceutical composition for the treatment, prevention and/or prophylaxis of adverse effects of glucocorticoid receptor agonist treatment or therapy.

In another embodiment of the present invention, the route of administration may be any route which effectively transports a compound according to the invention to the appropriate or desired site of action, such as oral, nasal, buccal, transdermal, pulmonal, or parenteral.

In still a further aspect of the invention the present compounds are administered in combination with one or more further active substances in any suitable ratios. Such further active substances may e.g. be selected from antiobesity agents, antidiabetics, agents modifying the lipid metabolism, antihypertensive agents, glucocorticoid receptor agonists, agents for the treatment and/or prevention of complications resulting from or associated with diabetes and agents for the treatment and/or prevention of complications and disorders resulting from or associated with obesity.

Thus, in a further aspect of the invention the present compounds may be administered in combination with one or more antiobesity agents or appetite regulating agents.

Such agents may be selected from the group consisting of CART (cocaine amphetamine regulated transcript) agonists, NPY (neuropeptide Y) antagonists, MC4 (melano-cortin 4) agonists, orexin antagonists, TNF (tumor necrosis factor) agonists, CRF (corticotropin releasing factor) agonists, CRF BP (corticotropin releasing factor binding protein) antagonists, urocortin agonists, β3 agonists, MSH (melanocyte-stimulating hormone) agonists, MCH (melanocyte-concentrating hormone) antagonists, CCK (cholecystokinin) agonists, serotonin re-uptake inhibitors, serotonin and noradrenaline re-uptake inhibitors, mixed serotonin and noradrenergic compounds, 5HT (serotonin) agonists, bombesin agonists, galanin antagonists, growth hormone, growth hormone releasing compounds, TRH (thyreotropin releasing hormone) agonists, UCP 2 or 3 (uncoupling protein 2 or 3) modulators, leptin agonists, DA agonists (bromocriptin, doprexin), lipase/amylase inhibitors, PPAR (peroxisome proliferator-activated receptor) modulators, RXR (retinoid X receptor) modulators, TR β agonists, AGRP (Agouti related protein) inhibitors, H3 histamine antagonists, opioid antagonists (such as naltrexone), exendin-4, GLP-1 and ciliary neurotrophic factor.

In one embodiment of the invention the antiobesity agent is leptin; dexamphetamine or amphetamine; fenfluramine or dexfenfluramine; sibutramine; orlistat; mazindol or phentermine.

Suitable antidiabetic agents include insulin, insulin analogues and derivatives such as those disclosed in EP 792 290 (Novo Nordisk A/S), e.g. N^{εB29}-tetradecanoyl des (B30) human insulin, EP 214 826 and EP 705 275 (Novo Nordisk A/S), e.g. Asp^{B28} human insulin, US 5,504,188 (Eli Lilly), e.g. Lys^{B28} Pro^{B29} human insulin, EP 368 187 (Aventis), eg Lantus, which are all incorporated herein by reference, GLP-1 (glucagon like peptide-1) and GLP-1 derivatives such as those disclosed in WO 98/08871 to Novo Nordisk A/S, which is incorporated herein by reference as well as orally active hypoglycaemic agents.

The orally active hypoglycaemic agents preferably comprise sulphonylureas, biguanides, meglitinides, glucosidase inhibitors, glucagon antagonists such as those disclosed in WO 99/01423 to Novo Nordisk A/S and Agouron Pharmaceuticals, Inc., GLP-1 agonists, potassium channel openers such as those disclosed in WO 97/26265 and WO 99/03861 to Novo Nordisk A/S, DPP-IV (dipeptidyl peptidase-IV) inhibitors, inhibitors of hepatic enzymes involved in stimulation of gluconeogenesis and/or glycogenolysis, glucose uptake modulators, compounds modifying the lipid metabolism such as antihyperlipidemic agents and antilipidemic agents as PPARα modulators, PPARδ modulators, cholesterol absorption inhibitors, HSL (hormone-sensitive lipase) inhibitors and HMG CoA inhibitors (statins), nicotinic acid, fibrates, anion exchangers, compounds lowering food intake, bile acid resins, RXR agonists and agents acting on the ATP-dependent potassium channel of the β-cells.

In one embodiment, the present compounds are administered in combination with insulin or an insulin analogue or derivative, such as N^{εB29}-tetradecanoyl des (B30) human insulin, Asp^{B28} human insulin, Lys^{B28} Pro^{B29} human insulin, Lantus®, or a mix-preparation comprising one or more of these.

In a further embodiment the present compounds are administered in combination with a sulphonylurea e.g. tolbutamide, glibenclamide, glipizide or glicazide.

In another embodiment the present compounds are administered in combination with a biguanide e.g. metformin.

In yet another embodiment the present compounds are administered in combination with a meglitinide e.g. repaglinide or senaglinide.

In still another embodiment the present compounds are administered in combination with a thiazolidinedione e.g. troglitazone, ciglitazone, pioglitazone, rosiglitazone or compound disclosed in WO 97/41097 such as 5-1[4-[3-Methyl-4-oxo-3,4-dihydro-2-quinazolinyl]methoxy]phenyl-methyl]thiazolidine-2,4-dione or a pharmaceutically acceptable salt thereof, preferably the potassium salt.

In yet another embodiment the present compounds may be administered in combination with the insulin sensitizers disclosed in WO 99/19313 such as (-) 3-[4-[2-Phenoxazin-10-yl)ethoxy]phenyl]-2-ethoxypropanoic acid or a pharmaceutically acceptable salts thereof, preferably the arginine salt.

In a further embodiment the present compounds are administered in combination with an α-glucosidase inhibitor e.g. miglitol or acarbose.

In another embodiment the present compounds are administered in combination with an, agent acting on the ATP-dependent potassium channel of the β-cells e.g. tolbutamide, glibenclamide, glipizide, glicazide or repaglinide.

Furthermore, the present compounds may be administered in combination with nateglinide.

In still another embodiment the present compounds are administered in combination with an antihyperlipidemic agent or antilipidemic agent e.g. cholestyramine, colestipol, clofibrate, gemfibrozil, fenofibrate, bezafibrate, tesaglitazar, EML-4156, LY-818, MK-767, atorvastatin, fluvastatin, lovastatin, pravastatin, simvastatin, acipimox, probucol, ezetimibe or dextrothyroxine.

In a further embodiment the present compounds are administered in combination with more than one of the above-mentioned compounds e.g. in combination with a sulphonylurea and metformin, a sulphonylurea and acarbose, repaglinide and metformin, insulin and a sulphonylurea, insulin and metformin, insulin, insulin and lovastatin, etc.

Further, the present compounds may be administered in combination with one or more antihypertensive agents. Examples of antihypertensive agents are β-blockers such as alprenolol, atenolol, timolol, pindolol, propranolol, metoprolol, bisoprololfumerate, esmolol, acebutelol, metoprolol, acebutolol, betaxolol, celiprolol, nebivolol, tertatolol, oxprenolol, amusolalul, carvedilol, labetalol, β2-receptor blockers e.g. S-atenolol, OPC-1085, ACE (angiotensin converting enzyme) inhibitors such as quinapril, lisinopril, enalapril, captopril, benazepril, perindopril, trandolapril, fosinopril, ramipril, cilazapril, delapril, imidapril, moexipril, spirapril, temocapril, zofenopril, S-5590, fasidotril, Hoechst-Marion Roussel: 100240 (EP 00481522), omapatrilat, gemopatrilat and GW-660511, calcium channel blockers such as nifedipine, felodipine, nicardipine, isradipine, nimodipine, diltiazem, amlodipine, nitrendipine, verapamil, lacidipine, lercanidipine, aranidipine, cilnidipine, clevidipine, azelnidipine, barnidipine, efonodipine, iasidipine, iemildipine, iercanidipine, manidipine, nilvadipine, pranidipine, furnidipine, α-blockers such as doxazosin, urapidil, prazosin, terazosin, bunazosin and OPC-28326, diuretics such as thiazides/sulphonamides (e.g. bendroflumetazide, chlorothalidone, hydrochlorothiazide and clopamide), loop-diuretics (e.g. bumetanide, furosemide and torasemide) and potassium sparing diuretics (e.g. amiloride, spironolactone), endothelin ET-A antagonists such as ABT-546, ambrisetan, atrasentan, SB-234551, Cl-1034, S-0139 and YM-598, endothelin antagonists e.g. bosentan and J-104133, renin inhibitors such as aliskiren, vasopressin V1 antagonists e.g. OPC-21268, vasopressin V2 antagonists such as tolvaptan, SR-121463 and OPC-31260, B-type natriuretic peptide agonists e.g. Nesiritide, angiotensin 11 antagonists such as irbesartan, candesartancilexetil, losartan, valsartan, telmisartan, eprosartan, candesartan, CL-329167, eprosartan, iosartan, olmesartan, pratosartan, TA-606, and YM-358, 5-HT2 agonists e.g. fenoldopam and ketanserin, adenosine A1 antagonists such as naftopidil, N-0861 and FK-352, thromboxane A2 antagonists such as KT2-962, endopeptidase inhibitors e.g. ecadotril, nitric oxide agonists such as LP-805, dopamine D1 antagonists e.g. MYD-37, dopamine D2 agonists such as nolomirole, n-3 fatty acids e.g. omacor, prostacyclin agonists such as treprostinil, beraprost, PGE1 agonists e.g. ecraprost, Na+/K+ ATPase modulators e.g. PST-2238, Potassium channel activators e.g. KR-30450, vaccines such as PMD-3117, Indapamides, CGRP-unigene, guanylate cyclase stimulators, hydralazines, methyldopa, docarpamine, moxonidine, CoAprovel, MondoBiotech-811.

Further reference can be made to Remington: The Science and Practice of Pharmacy, 19th Edition, Gennaro, Ed., Mack Publishing Co., Easton, PA, 1995.

Furthermore, the present compounds may be administered in combination with one or more glucocorticoid receptor agonists. Examples of such glucocorticoid receptor agonists are betametasone, dexamethasone, hydrocortisone, methylprednisolone, prednisolone, prednisone, beclomethasone, butixicort, clobetasol, flunisolide, flucatisone (and analogues), momethasone, triamcinolonacetonide, triamcinolonhexacetonide GW-685698, NXC-1015, NXC-1020, NXC-1021, NS-126, P-4112, P-4114, RU-24858 and T-25 series.

It should be understood that any suitable combination of the compounds according to the invention with one or more of the above-mentioned compounds and optionally one or more further pharmacologically active substances are considered to be within the scope of the present invention.

### PHARMACEUTICAL COMPOSITIONS

The compounds of the present invention may be administered alone or in combination with pharmaceutically acceptable carriers or excipients, in either single or multiple doses. The pharmaceutical compositions according to the invention may be formulated with pharmaceutically acceptable carriers or diluents as well as any other known adjuvants and excipients in accordance with conventional techniques such as those disclosed in Remington: The Science and Practice of Pharmacy,19th Edition, Gennaro, Ed., Mack Publishing Co., Easton, PA, 1995.

The pharmaceutical compositions may be specifically formulated for administration by any suitable route such as the oral, rectal, nasal, pulmonary, topical (including buccal and sublingual), transdermal, intracisternal, intraperitoneal, vaginal and parenteral (including subcutaneous, intramuscular, intrathecal, intravenous and intradermal) route, the oral route being preferred. It will be appreciated that the preferred route will depend on the general condition and age of the subject to be treated, the nature of the condition to be treated and the active ingredient chosen.

Pharmaceutical compositions for oral administration include solid dosage forms such as hard or soft capsules, tablets, troches, dragees, pills, lozenges, powders and granules. Where appropriate, they can be prepared with coatings such as enteric coatings or they can be formulated so as to provide controlled release of the active ingredient such as sustained or prolonged release according to methods well-known in the art.

Liquid dosage forms for oral administration include solutions, emulsions, suspensions, syrups and elixirs.

Pharmaceutical compositions for parenteral administration include sterile aqueous and non-aqueous injectable solutions, dispersions, suspensions or emulsions as well as sterile powders to be reconstituted in sterile injectable solutions or dispersions prior to use. Depot injectable formulations are also contemplated as being within the scope of the present invention.

Other suitable administration forms include suppositories, sprays, ointments, crèmes, gels, inhalants, dermal patches, implants etc.

A typical oral dosage is in the range of from about 0.001 to about 100 mg/kg body weight per day, preferably from about 0.01 to about 50 mg/kg body weight per day, and more preferred from about 0.05 to about 10 mg/kg body weight per day administered in one or more dosages such as 1 to 3 dosages. The exact dosage will depend upon the frequency and mode of administration, the sex, age, weight and general condition of the subject treated, the nature and severity of the condition treated and any concomitant diseases to be treated and other factors evident to those skilled in the art.

The formulations may conveniently be presented in unit dosage form by methods known to those skilled in the art. A typical unit dosage form for oral administration one or more times per day such as 1 to 3 times per day may contain from 0.05 to about 2000 mg, e.g. from about 0.1 to about 1000 mg, from about 0.5 mg to about 500 mg., from about 1 mg to about 200 mg, e.g. about 100 mg.

For parenteral routes, such as intravenous, intrathecal, intramuscular and similar administration, typically doses are in the order of about half the dose employed for oral administration.

The compounds of this invention are generally utilized as the free substance or as a pharmaceutically acceptable salt thereof. Examples are an acid addition salt of a compound having the utility of a free base and a base addition salt of a compound having the utility of a free acid. The term "pharmaceutically acceptable salts" refers to non-toxic salts of the compounds for use according to the present invention which are generally prepared by reacting the free base with a suitable organic or inorganic acid or by reacting the acid with a suitable organic or inorganic base. When a compound for use according to the present invention, contains a free base such salts are prepared in a conventional manner by treating a solution or suspension of the compound with a chemical equivalent of a pharmaceutically acceptable acid. When a compounds for use according to the present invention, contains a free acid such salts are prepared in a conventional manner by treating a solution or suspension of the compound with a chemical equivalent of a pharmaceutically acceptable base. Physiologically acceptable salts of a compound with a hydroxy group include the anion of said compound in combination with a suitable cation such as sodium or ammonium ion. Other salts which are not pharmaceutically acceptable may be useful in the preparation of compounds for use according to the present invention and these form a further aspect of the present invention.

For parenteral administration, solutions of the present compounds in sterile aqueous solution, aqueous propylene glycol or sesame or peanut oil may be employed. Such aqueous solutions should be suitable buffered if necessary and the liquid diluent first rendered isotonic with sufficient saline or glucose. The aqueous solutions are particularly suitable for intravenous, intramuscular, subcutaneous and intraperitoneal administration. The sterile aqueous media employed are all readily available by standard techniques known to those skilled in the art.

Suitable pharmaceutical carriers include inert solid diluents or fillers, sterile aqueous solution and various organic solvents. Examples of suitable carriers are water, salt solutions, alcohols, polyethylene glycols, polyhydroxyethoxylated castor oil, peanut oil, olive oil, syrup, phospholipids, gelatine, lactose, terra alba, sucrose, cyclodextrin, amylose, magnesium stearate, talc, gelatin, agar, pectin, acacia, stearic acid or lower alkyl ethers of cellulose, silicic acid, fatty acids, fatty acid amines, fatty acid monoglycerides and diglycerides, pentaerythritol fatty acid esters, polyoxyethylene, hydroxymethylcellulose and polyvinylpyrrolidone. Similarly the carrier or diluent may include any sustained release material known in the art, such as glyceryl monostearate or glyceryl distearate, alone or mixed with a wax. The formulations may also include wetting agents, emulsifying and suspending agents, preserving agents, sweetening agents or flavouring agents.

The pharmaceutical compositions formed by combining the compounds of the invention and the pharmaceutically acceptable carriers are then readily administered in a variety of dosage forms suitable for the disclosed routes of administration. The formulations may conveniently be presented in unit dosage form by methods known in the art of pharmacy.

Formulations of the present invention suitable for oral administration may be presented as discrete units such as capsules or tablets, each containing a predetermined amount of the active ingredient, and which may include a suitable excipient. These formulations may be in the form of powder or granules, as a solution or suspension in an aqueous or non-aqueous liquid, or as an oil-in-water or water-in-oil liquid emulsion.

Compositions intended for oral use may be prepared according to any known method, and such compositions may contain one or more agents selected from the group consisting of sweetening agents, flavouring agents, colouring agents, and preserving agents in order to provide pharmaceutically elegant and palatable preparations. Tablets may contain the active ingredient in admixture with non-toxic pharmaceutically-acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example corn starch or alginic acid; binding agents, for example, starch, gelatine or acacia; and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the techniques described in U.S. Patent Nos. 4,356,108; 4,166,452; and 4,265,874, to form osmotic therapeutic tablets for controlled release.

Formulations for oral use may also be presented as hard gelatine capsules where the active ingredient is mixed with an inert solid diluent, for example, calcium carbonate, calcium phosphate or kaolin, or a soft gelatine capsule wherein the active ingredient is mixed with water or an oil medium, for example peanut oil, liquid paraffin, or olive oil.

Aqueous suspensions may contain the active compounds in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide such as lecithin, or condensation products of an alkylene oxide with fatty acids, for example polyoxyethylene stearate, or condensation products of ethylene oxide with long chain aliphatic alcohols, for example, heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more colouring agents, one or more flavouring agents, and one or more sweetening agents, such as sucrose or saccharins.

Oily suspensions may be formulated by suspending the active ingredient in a vegetable oil, for example arachis oil, olive oil, sesame oil or coconut oil, or in a mineral oil such as a liquid paraffin. The oily suspensions may contain a thickening agent, for example beeswax, hard paraffin or cetyl alcohol. Sweetening agents such as those set forth above, and flavouring agents may be added to provide a palatable oral preparation. These compositions may be preserved by the addition of an anti-oxidant such as ascorbic acid.

Dispersible powders and granules suitable for preparation of an aqueous suspension by the addition of water provide the active compound in admixture with a dispersing or wetting agent, suspending agent and one or more preservatives. Suitable dispersing or wetting agents and suspending agents are exemplified by those already mentioned above. Additional excipients, for example, sweetening, flavouring, and colouring agents may also be present.

The pharmaceutical compositions comprising a compound for use according to the present invention may also be in the form of oil-in-water emulsions. The oily phase may be a vegetable oil, for example, olive oil or arachis oil, or a mineral oil, for example a liquid paraffin, or a mixture thereof. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of said partial esters with ethylene oxide, for example polyoxyethylene sorbitan monooleate. The emulsions may also contain sweetening and flavouring agents.

Syrups and elixirs may be formulated with sweetening agents, for example glycerol, propylene glycol, sorbitol or sucrose. Such formulations may also contain a demulcent, preservative and flavouring and colouring agent. The pharmaceutical compositions may be in the form of a sterile injectable aqueous or oleaginous suspension. This suspension may be formulated according to the known methods using suitable dispersing or wetting agents and suspending agents described above. The sterile injectable preparation may also be a sterile injectable solution or suspension in a non-toxic parenterally-acceptable diluent or solvent; for example as a solution in 1,3-butanediol. Among the acceptable vehicles and solvents that may be employed are water, Ringer's solution, and isotonic sodium chloride solution. In addition, sterile, fixed oils are conveniently employed as solvent or suspending medium. For this purpose, any bland fixed oil may be employed using synthetic mono- or diglycerides. In addition, fatty acids such as oleic acid find use in the preparation of injectables.

The compositions may also be in the form of suppositories for rectal administration of the compounds of the present invention. These compositions can be prepared by mixing the drug with a suitable non-irritating excipient which is solid at ordinary temperatures but liquid at the rectal temperature and will thus melt in the rectum to release the drug. Such materials include cocoa butter and polyethylene glycols, for example.

For topical use, creams, ointments, jellies, solutions of suspensions, etc., containing the compounds of the present invention are contemplated. For the purpose of this application, topical applications shall include mouth washes and gargles.

The compounds for use according to the present invention may also be administered in the form of liposome delivery systems, such as small unilamellar vesicles, large unilamellar vesicles, and multilamellar vesicles. Liposomes may be formed from a variety of phospholipids, such as cholesterol, stearylamine, or phosphatidylcholines.

In addition, some of the compounds for use according to the present invention may form solvates with water or common organic solvents. Such solvates are also encompassed within the scope of the present invention.

Thus, in a further embodiment, there is provided a pharmaceutical composition comprising a compound for use according to the present invention, or a pharmaceutically acceptable salt, solvate, or prodrug thereof, and one or more pharmaceutically acceptable carriers, excipients, or diluents.

If a solid carrier is used for oral administration, the preparation may be tabletted, placed in a hard gelatine capsule in powder or pellet form or it can be in the form of a troche or lozenge. The amount of solid carrier-will vary widely but will usually be from about 25 mg to about 1 g. If a liquid carrier is used, the preparation may be in the form of a syrup, emulscion, soft gelatine capsule or sterile injectable liquid such as an aqueous or non-aqueous liquid suspension or solution.

A typical tablet which may be prepared by conventional tabletting techniques may contain:

| Core: | |
|---|---|
| Active compound (as free compound or salt thereof) | 5.0 mg |
| Lactosum Ph. Eur. | 67.8 mg |
| Cellulose, microcryst. (Avicel) | 31.4 mg |
| Amberlite^{®}IRP88* | 1.0 mg |
| Magnesii stearas Ph. Eur. | q.s. |
| | |

| Coating: | |
|---|---|
| Hydroxypropyl methylcellulose | approx. 9 mg |
| Mywacett 9-40 T** | approx. 0.9 mg |

| | |
|---|---|
| * Polacrillin potassium NF, tablet disintegrant, Rohm and Haas. ** Acylated monoglyceride used as plasticizer for film coating. | |

The compounds of the invention may be administered to a patient which is a mammal, especially a human in need thereof. Such mammals include also animals, both domestic animals, e.g. household pets, and non-domestic animals such as wildlife.

The present invention also relate to the below methods of preparing the compounds of the invention.

The present invention is further illustrated in the following representative examples

**Microwave oven synthesis:** The reaction was heated by microwave irradiation in sealed microwave vessels in a single mode Emrys Optimizer EXP from PersonalChemistry®.

**Preparative HPLC: Column:** 1.9 x 15 cm Waters XTerra RP-18. Buffer linear gradient 5 - 95 % in 15 min, MeCN, 0:1 % TFA, flow rate of 15 ml/min. The pooled fractions are either evaporated to dryness *in vacuo,* or evaporated *in vacuo* until the MeCN is removed, and then frozen and freeze dried.

### Reference Example 1 (General method (A)), Formula (I)

### 4-(Benzo[1,3]dioxol-5-yioxy)-N-cyclohexyl-N-methyl-butyramide

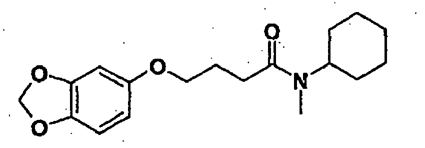

To a solution of 4-(benzo[1,3]dioxol-5-yloxy)-butyric acid (1.0 g, 4.46 mmol), HOBT (0.66 g, 4.91 mmol) in dry THF (75 ml) was added EDAC (0.94 g, 4.91 mmol) and the mixture was stirred for 20 minutes. Di-isopropyl ethyl amine (DIPEA) (860 µl, 4.91 mmol) and cyclohexyl-methyl-amine (555 mg, 4.91 mmol) was added and the resulting mixture was stirred for 16 hrs. at room temperature. The volatiles were evaporated *in vacuo* and the residue was purified by silicagel chromatography using a mixture of ethyl acetate/hexane (1:4) as eluent. Pure fractions were collected and the solvent evaporated *in vacuo* to dryness. The olly residue crystallized on standing affording 1.1 g (77%) of the title compounds as a solid.
¹H NMR (300 MHz, CDCl₃) δ1.07-1.85 (m, 11H), 2.10 (m, 2H), 2.48 (t, 1H), 2.53 (t, 1H), 2.81 and 2.83 (2x s, 3H, N-Me rotamers), 3.96 (t, 2H), 5.90 (s, 2H), 6.32 (dd, 1H), 6.49 (d, 1H), 6.69 (d, 1H).
Calculated for G₁₈H₂₅NO₄₃
C, 67.69 %; H, 7.89 %; N,4.39%. Found:
C, 67.74 %; H, 7.99 %; N,4.34%.

### Reference Example 2 (General method (A)), Fomula (I)

### N-Methyl-N-(1-methyl-piperidin-4-yl)-4-phenoxy-butyramide

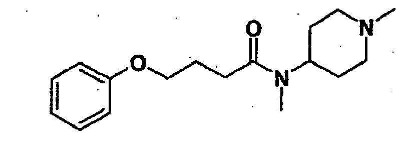

To a solution of 4-phenoxy-butyric acid (1.0 g, 5.55 mmol), HOBT (0.83 g, 6.1 mmol) in dry THF (75 ml) was added EDAC (1.17 g, 6.1 mmol) and the mixture was stirred for 20 minutes. Di-isopropyl ethyl amine (DIPEA) (1.06 ml, 6.1 mmol) and methyl-(1-methyl-piperidin-4-yl)-amine (783 mg, 6.1 mmol) was added and the resulting mixture was stirred for 16 hrs. at room temperature. The volatiles were evaporated *in vacuo* and the residue was purified by silicagel chromatography using a mixture of ethyl acetate/triethyl amine (1:25) as eluent. Pure fractions were collected and the solvent evaporated *in vacuo* to dryness affording 1.1 g (68%) of the title compounds as an oil.
¹H NMR (300 MHz, CDCl₃) δ 1.57 (m, 2H), 1.74 (dq, 1H), 1.86-2.18 (m, 5H), 2.28 (t, 3H), 2.52 (m, 2H), 2.82 and 2.85 (2x s, 3H, N-Me rotamers), 2.89 (bd, 2H), 3.60 and 4.51 (2x dt,
1H), 4.04 (t, 2H), 6.91 (m, 3H), 7.28 (m, 2H). Calculated for C₁₇H₂₆N₂O₂;
C, 70.31 %; H, 9.02 %; N, 9.65 %. Found:
C, 69.72 %; H, 9.29 %; N, 10.12 %.

### EXAMPLES, COMPOUNDS OF GENERAL FORMULA (III)

The following examples and general procedures refer to intermediate compounds and final products for general formula (III) identified in the specification and in the synthesis schemes. The preparation of the compounds of general formula (III) of the present Invention is described in detail using the following examples. Occasionally, the reaction may not be applicable as described to each compound included within the disclosed scope of the invention. The compounds for which this occurs will be readily recognised by those skilled in the art. In these cases the reactions can be successfully performed by conventional modifications known to those skilled in the art, that is, by appropriate protection of interfering groups, by changing to other conventional reagents, or by routine modification of reaction conditions. Alternatively, other reactions disclosed herein or otherwise conventional will be applicable to the preparation of the corresponding compounds of the invention. In all preparative methods, all starting materials are known or may easily be prepared from known starting materials. The structures of the compounds are confirmed by either elemental analysis or nuclear magnetic resonance (NMR), where peaks assigned to characteristic protons in the title compounds are presented where appropriate. ¹H NMR shifts (δ_{H}) are given in parts per million (ppm) down field from tetramethylsilane as internal reference standard. M.p.: is melting point and is given in °C and is not corrected. Column chromatography was carried out using the technique described by W.C. Still et al., J. Org. Chem. 43: 2923 (1978) on Merck silica gel 60 (Art. 9385). HPLC analyses are performed using 5µm C18 4 x 250 mm column eluted with various mixtures of water and acetonitrile, flow = 1 ml/min, as described in the experimental section.

The abbreviations as used in the examples have the following meaning:
- TLC:: thin layer chromatography
- CDCl₃:: deuterio chloroform
- CD₃OD:: tetradeuterio methanol
- DMSO-d₆:: hexadeuterio dimethylsulfoxide
- DMSO:: dimethylsulfoxide
- THF:: tetrahydrofuran
- DMF:: N,N-dimethylformamide
- HOBT:: 1-hydroxy-benzotriazole
- EDAC:: 1-(3-dimethylaminopropyl)-3-ethylcarbodiimide, hydrochloride
- min:: minutes
- hrs:: hours

The compounds of the invention are prepared as illustrated in the following reaction scheme 1:

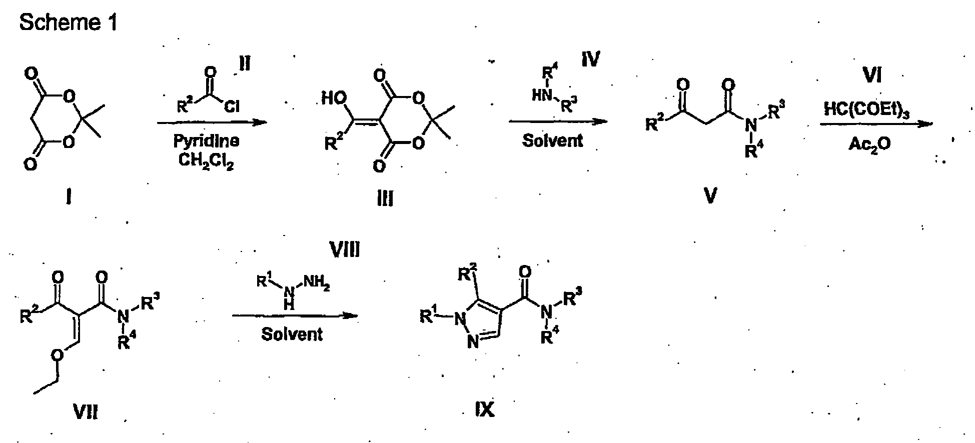

### General method:

By allowing 2,2-dimethyl-1,3-dioxane-4,6-dione (Meldrum's acid) (I) to react with an acid chloride (11), wherein R² is defined above, in a solvent mixture of pyridine and DCM-and affording an acyl Meldrum's acid (III), wherein R² is as defined above. The acyl Meldrum's acid (III) is aminolysed with an substituted amine (IV), wherein R³ and R⁴ are defined above, in a solvent such as benzene, toluene, dioxane, at a temperature interval from 50 °C up to reflux affording a *beta*-keto amide (V) wherein R², R³ and R⁴ are as defined above. The *beta*-keto amide (V) is treated with *ortho*-formiate (VI) in a solvent such as acetic acid anhydride at a temperature Interval from 50 °C up to reflux affording enol ether (VII) wherein R², R³, and R⁴ are as defined above. Condensation of the enol ether (VII) wherein R², R³, and R⁴ are as defined above with hydrazide (VIII) wherein R¹ is as defined above yields pyrazole (IX) wherein R¹, R², R³, and R⁴ are as defined above, in a solvent such as EtOH, *i*-PrOH, *tert*-BuOH.

### Example 1

### 1-(4-Chloro-ohenyl)-5-propyl-1H-pyrazole-4-carboxylic acid cyclohexyl-methyl-amide

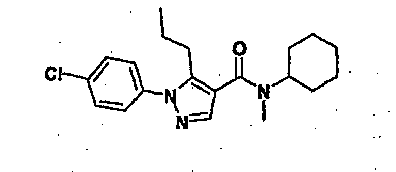

To a solution of 1-(4-chloro-phenyl)-5-propyl-1*H-*pyrazole-4-carboxylic acid (1.0 g, 3.78 mmol), HOBT (0.56 g, 4.16 mmol) in dry THF (50 ml) was added EDAC (0.8 g, 4.16 mmol) and the mixture was stirred for 10 minutes. Di-isopropyl ethyl amine (DIPEA) (724 µl, 4.16 mmol) and cyclohexyl-methyl-amine (0.54 ml, 4.16 mmol) was added and the resulting mixture was stirred for 16 hrs. at room temperature. The volatiles were evaporated *in vacuo* and the residue was purified by silicagel chromatography using a mixture of ethyl acetate and heptane (1:4) as eluent. Pure fractions were collected and the solvent evaporated *in vacuo* affording 1.1 g (81%) of the title compounds as an oil.
¹H NMR (300 MHz, CDCl₃) δ 0.81 (t, 3H), 1.1 -1.86 (m, 13H), 2.81 (t, 2H), 2.98 (s, 3H), 3.89 and 4.50 (2 x bs, 1H), 7.37 (m, 2H), 7.47 (m, 2H), 7.58 (bs, 1H).
Calculated for C₂₀H₂₆ClN₃O, 0.4 H₂O;
C, 65.44 %; H, 7.36 %; N, 11.45 %. Found:
C, 65.47 %; H, 7.89 %; N, 11.56 %.

The following compound was made in a similar way as described in example 1 above

### Example 2

### 1-(4-Chloro-phenyl)-5-trifluoromethyl-1H-pyrazole-4-carboxylic acid cyclohexyl-methyl-amide

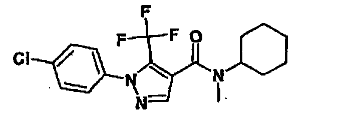

Calculated for C₁₈H₁₉ClF₃N₃O;
C, 56.04 %; H, 4.96 %; N, 10.89 %. Found:
C, 56.02 %; H, 5.1 6 %; N, 10.76 %.

### Example 3

### [1-(4-Methoxy-phenyl)-5-methyl-1H-pyrazol-4-yl]-(1,3,3-trimethyl-6-aza-bicyclo[3.2.1]oct-6-yl)-methanone

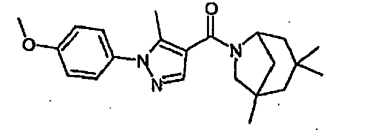

¹H NMR (400 MHz, CDCl₃) δ 0.97 (t, 5H), 1.11 (t, 4H), 1.25 -1.61 (m, 4H), 1.79 (m, 2H), 2.20 (d, 0.5H), 2.44 (d, 3H), 3.29 (d, 0.5H), 3.40 (d, 0.5H), 3.59 (m, 0.5H), 3.86 (s, 3H), 4.37 (m, 0.5H), 4.60 (m, 0.5H), 6.99 (d, 2H), 7.33 (d, 2H), 7.74 (s, 1H).

### Reference Example 1, Formula (V)

### 1H-Benzoimidazole-5-carboxylic acid cyclohexyl-methyl-amide

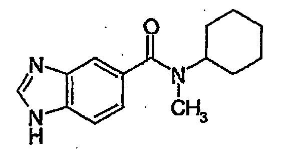

A solution of 1*H*-benzoimidazoie-5-carboxylic acid (10 g, 61.67 mmol) and HOBT (9.17 g, 67.84 mmol) in dry THF (250 ml) was stirred for 1 h. EDAC (13 g, 67.84 mmol) was added and the mixture was stirred for another 1 h. Di-isopropyl ethyl amine (DIPEA) (11.8 ml, 67.84 mmol) and cyclohexyl-methyl-amine (8.8 ml, 67.84 mmol) was added and the resulting mixture was stirred for 16 hrs. at room temperature. The precipitate was filtered off and the volatiles were evaporated *in vacuo.* To the residue was added water (150 ml) and diethyl ether (75 ml) and the resulting mixture was stirred for 15 minutes. The precipitate was filtered off and washed with water followed by diethyl ether and drying *in vacuo* at 50 °C which afforded 7.7 g (49%) of the title compounds as a solid.
¹H NMR (300 MHz, CDCl₃) δ 1.03 (bs, 2H), 1.53 - 1.86 (m, 8H), 2.93 (bd, 3H), 3.57 and 4.56 (2 x bs, 1H), 7.19 (d, 1H), 7.44 (bs, 1H), 7.62 (s, 1H), 7.79 (s, 1H), 11.8 (bs, 1H, N*H*).
Calculated for C₁₅H₁₉N₃O;
C, 70.01 %; H, 7.44 %; N, 16.33 %. Found:
C, 69.63 %; H, 7.45 %; N, 16.17 %.

### Reference Example 1 (General method B), Formula (VI)

### 2,3-Dimethyl-2,3-dihydro-benzofuran-7-carboxylic acid cyclohexyl-methyl-amide

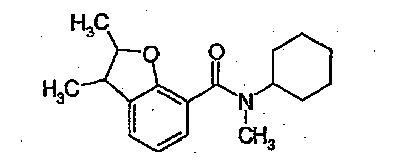

To a solution of 2,3-dimethyl-2,3-dihydro-benzofuran-7-carboxylic acid (0.5 g, 2.60 mmol), HOBT (0.39 g, 2.86 mmol) In dry THF (25 ml) was added EDAC (0.55 g, 2.86 mmol). The mixture was stirred for 10 min. followed by addition of di-isopropyl ethyl amine (DIPEA) (0.5 ml, 2.86 mmol) and cyclohexyl-methyl-amine (0.37 ml, 2.86 mmol). The resulting mixture was stirred for 16 hrs. at room temperature, the volatiles were evaporated *in vacuo* and to the residue was added water (25 ml) and diethyl ether (75 ml). The organic phase was separated and dried (Na₂SO₄), filtered and the solvent evaporated *in vacuo.* The residue was dissolved in a mixture of AcOEt/Heptane (1:1) and filtered through a 2.5 cm silicagel plug. The solvent was evaporated *in vacuo* affording 0.7 g (93%) of the title compounds as an oil.
¹H NMR (300 MHz, CDCl₃) δ 1.06 (m, 3H), 1.29 (m, 4H), 1.48 (m, 5H), 1.67 -1.8 (m, 4H), 2.77 and 2.97 (d and s, 3H, rotamer), 3.05 and 4.37 (2 x m, 1H), 3.35 and 4.55 (2 x m, 1H), 3.39 and 4.89 (2 x m, 1H), 6.86 (t, 1H), 7.12 (t, 2H).
Calculated for C₁₈H₂₅NO₂, 0.15 H₂O
C, 74.52 %; H, 8.79 %; N, 4.83 %. Found:
C, 74.54 %; H, 8.98 %; N, 4.98 %.

### Reference Example 2, Formula (VII)

### 1H-Indole-6-carboxylic acid cyclohexyl-methyl-amide

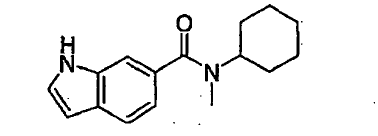

A solution of 1*H*-indole-6-carboxylic acid (1.0 g, 6.21 mmol), HOBT (0.92 g, 6.83 mmol) in dry THF (50 mL) and EDAC (1.31 g, 6.83 mmol) was stirred for 20 mins. Di-isopropyl ethyl amines (DIPEA) (1.2 mL, 6.83 mmol) and cyclohexyl-methyl-amine (0.9 mL, 6.83 mmol) was added and the resulting mixture was stirred for 16 hrs. at room temperature. To the precipitate was added water (50 mL), the solid filtered off, washed with water followed by diethyl ether and dried *in vacuo* at 50 °C which afforded 0.6 g (38%) of the title compounds as a solid.
¹H NMR (300 MHz, CDCl₃) δ 1.05 (bm, 2H), 1.15 - 1.85 (m, 8H), 2.96 (bs, 3H), 3.65 and 4.55 (2x m, 1H), 6.54 (s, 1H), 7.09 (d, 1H), 7.24 (m, 1H), 7.50 (s, 1H), 7.60 (d, 1H), 8.89 (bs, 1H, N*H*).
Calculated for C₁₈H₂₀N₂O;
C, 74.97 %; H, 7.86 %; N, 10.93 %. Found;C, 74.90 %; H, 8.01 %; N, 10.88 %.

### PHARMACOLOGICAL METHODS

### 11βHSD1 enzyme assay

### Materials

³H-cortisone and anti-rabbit Ig coated scintillation proximity assay (SPA) beads were purchased from Amersham Pharmacia Biotech, β-NADPH was from Sigma and rabbit anti-cortisol antibodies were from Fitzgerald. An extract of yeast transformed with h-11βHSD1 (Hult et al., FEBS Lett., 441, 25 (1998)) was used as the source of enzyme. The test compounds were dissolved in DMSO (10 mM). All dilutions were performed in a buffer containing 50 mM TRIS-HCI (Sigma Chemical Co), 4 mM EDTA (Sigma Chemical Co), 0.1 % BSA (Sigma Chemical Co), 0.01% Tween-20 (Sigma Chemical Co) and 0.005% bacitracin (Novo Nordisk A/S), pH=7.4. Optiplate 96 wells plates were supplied by Packard. The amount of ³H-cortisol bound to the SPA beads was measured on TopCount NXT, Packard.

### Methods

h-11βHSD1, 120 nM ³H-cortisone, 4 mM β-NADPH, antibody (1:200), serial dilutions of test compound and SPA particles (2 mg/well) were added to the wells. The reaction was Initiated by mixing the different components and was allowed to proceed under shaking for 60 min at 30°C. The reaction was stopped be the addition of 10 fold excess of a stopping buffer containing 500 µM carbenoxolone and 1 µM cortisone. Data was analysed using GraphPad Prism software.

**Table 1**

| Inhibition of 11βHSD1 by compounds of the invention | | |
|---|---|---|
| Formula | Example No. | 11βHSD1 IC₅₀ values |
| (I) * | 1 | 0.56 µM |
| (I) * | 2 | 120 µM |
| (III) | 1 | 0.04 µM |
| (V) * | 1 | 45 nM |
| (VI) * | 1 | 6nM |
| (VII) * | 2 | 118 nM |

| | | |
|---|---|---|
| * Reference | | |

## Claims

1. A compound of formula (III): 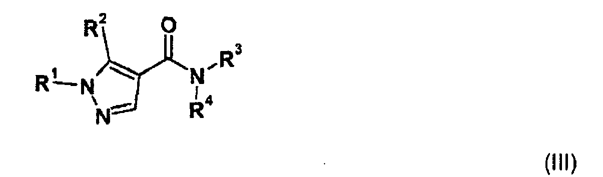 wherein
R¹ is aryl, arylC₁-C₆alkyl, hetaryl or hetarylC₁-C₆alkyl optionally substituted with one or more of R⁶ independently;
R² is halo, C₁-C₆alkyl, C₂-C₆alkenyl, C₂-C₆alkynyl, C₃C₁₀cycloalkyl, C₃-C₁₀cycloalkylC₁-C₆-alkyl, trihalomethyl, aryl, arylC₁-C₆alkyl, C₁-C₆alkyloxyC₁-C₆alkyl, arylC₁-C₆alkyloxyC₁-C₆alkyl, C₁-C₆alkylNR⁵C₁-C₆alkyl, arylC₁-C₆alkylNR⁵C₁-C₆alkyl, hetaryl or hetarylC₁-C₆alkyl wherein the alkyl, alkenyl, alkynyl, cycloalkyl and aryl groups independently are optionally substituted with one or more R⁷;
R³ is C₁-C₆alkyl optionally substituted with one or more of R⁸;
R⁴ is C₆-C₁₀cycloalkyl, C₆-C₁₀hetcycloalkyl, C₆-C₁₀cycloalkylC₁-C₆alkyl or C₆-C₁₀hetcycloalkylC₁-C₆alkyl wherein the alkyl, cycloalkyl and hetcycloalkyl groups independently are optionally substituted with one or more of R⁸; or
R³ and R⁴ together with the nitrogen to which they are attached, are forming a saturated or partially saturated bicyclic/bridge ring system containing from 7 to 12 carbon atoms and from 0 to 2 additional heteroatoms selected from nitrogen, oxygen or sulphur, the ring system optionally being substituted with at least one of C₁-C₆alkyl, aryl, hetaryl, arylC₁-C₆alkyl, hetaryl-C₁-C₆alkyl, hydroxy, oxo, C₁-C₆alkyloxy, arylC₁-C₆alkyloxy or hetarylC₁-C₆alkyloxy, wherein the alkyl and aryl groups independently are optionally substituted with one ore more of R⁹;
R⁵ is C₁-C₆alkyl, C₂-C₆alkenyl, C₂-C₆alkynyl, C₃-C₁₀cycloalkyl, C₃-C₁₀hetcycloalkyl, C₃-C₁₀-cycloalkylC₁-C₆alkyl, C₃-C₁₀hetcycloalkylC₁-C₆alkyl, aryl, hetaryl, arylC₁-C₆alkyl or hetarylC₁-C₆alkyl wherein the alkyl, alkenyl, alkynyl, aryl, hetaryl, cycloalkyl and hetcycloalkyl groups independently are optionally substituted with one or more of R⁹;
R⁶ and R⁷ independently are hydrogen, hydroxy, oxo, halo, nitro, cyano, C₁-C₆alkyl, C₁-C₆-alkyloxy, trihalomethyl, trihalomethoxy, NR¹⁰R¹¹, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, C₁-C₆alkyloxycarbonyl, aryloxycarbonyl, arylC₁-C₆alkyloxycarbonyl, C₁-C₆alkylcarboxy, arylcarboxy or arylC₁-C₆alkylcarboxy;
R⁸ and R⁹ independently are hydrogen, C₁-C₆alkyl, aryl, hetaryl, arylC₁-C₆alkyl, hetarylC₁-C₆-alkyl, hydroxy, oxo, cyano, NR¹⁰R¹¹, C₁-C₆alkyloxy, aryloxy, arylC₁-C₆alkyloxy, hetaryloxy, hetarylC₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-C₆alkylcarboxy;
R¹⁰ and R¹¹ independently are hydrogen, C₁-C₈alkyl, aryl, hetaryl, arylC₁-C₆alkyl, C₃-C₁₀-cycloalkyl, C₃-C₁₀hetcycloalkyl, C₃-C₁₀cycloalkylC₁-C₆, C₁-C₆alkylcarbonyl, C₁-C₆alkylcarboxyC₁-C₆alkyl; or
R¹⁰ and R¹¹ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₈alkyl , aryl, hetaryl, arylC₁-C₆alkyl, hetarylC₁-C₆alkyl, hydroxy, oxo, cyano, C₁-C₆alkyloxy, arylC₁-C₆alkyloxy, hetarylC₁-C₆alkyloxy, C₁-C₆alkyloxyC₁-C₆alkyl, C₁-C₆alkylcarbonyl, arylcarbonyl, hetarylcarbonyl, aryl-C₁-C₆alkylcarbonyl, hetarylC₁-C₆alkylcarbonyl, C₁-C₆alkylcarboxy, arylcarboxy or arylC₁-₆-alkylcarboxy; or
a salt thereof with a pharmaceutically acceptable acid or base, or any optical isomer or mixture of optical isomers, including a racemic mixture, or any tautomeric forms.

2. A compound according to claim 1 wherein R¹ is aryl or hetaryl optionally substituted with one or more R⁶ independently;
R² is halo, C₁-C₆alkyl, C₂-C₆alkenyl, C₂-C₆alkynyl, C₃-C₁₀cycloalkyl, C₃-C₁₀cycloalkylC₁-C₆-alkyl, aryl, arylC₁-C₆alkyl, C₁-C₆alkyloxyC₁-C₆alkyl, arylC₁-C₆alkyloxyC₁-C₆alkyl, C₁-C₆-alkylNR⁵C₁-C₆alkyl, arylC₁-C₆alkylNR⁵C₁-C₆alkyl, hetaryl or hetarylC₁-C₆alkyl wherein the alkyl, alkenyl, alkynyl, cycloalkyl and aryl groups independently are optionally substituted with one or more R⁷;
R³ is C₁-C₆alkyl optionally substituted with one or more of R⁸;
R⁴ is C₆-C₁₀cycloalkyl, C₆-C₁₀hetcycloalkyl, C₆-C₁₀cycloalkylC₁-C₆alkyl or C₆-C₁₀hetcycloalkylC₁-C₆alkyl wherein the alkyl, cycloalkyl and hetcycloalkyl groups independently are optionally substituted with one or more of R⁸;
R⁵ is C₁-C₆alkyl, C₂-C₆alkenyl, C₂-C₆alkynyl, C₃-C₁₀cycloalkyl, C₃-C₁₀hetcycloalkyl, C₃-C₁₀-cycloalkylC₁-C₆alkyl, C₃-C₁₀hetcycloalkylC₁C₆alkyl, aryl, hetaryl, arylC₁-C₆alkyl or hetarylC₁-C₆alkyl wherein the alkyl, alkenyl, alkynyl, aryl, hetaryl, cycloalkyl and hetcycloalkyl groups independently are optionally substituted with one or more of R⁹;
R⁶ and R⁷ independently are hydrogen, hydroxy, oxo, halo, nitro, cyano, C₁-C₆alkyl, C₁-C₆-alkyloxy, trihalomethyl, trihalomethoxy, NR¹⁰R¹¹, arylC₁-C₆alkyloxy, hetarylC₁-C₆alkyloxy, C₁-C₆alkylcarbonyl, arylcarbonyl, hetarylcarbonyl, arylC₁₋₆alkylcarbonyl, C₁-C₆alkyloxycarbanyl, aryloxycarbonyl, arylC₁-C₆alkyloxycarbonyl, C₁-C₆alkylcarboxy, arylcarboxy or arylC₁-C₆alkylcarboxy;
R⁸ and R⁹ independently are hydrogen, C₁-C₆alkyl, aryl, hetaryl, arylC₁-C₆alkyl, hetarylC₁-C₆-alkyl, hydroxy, oxo, cyano, NR¹⁰R¹¹, C₁-C₆alkyloxy, aryloxy, arylC₁-C₆alkyloxy, hetaryloxy, hetarylC₁-C₆alkyloxy, C₁-C₆alkyloxyC₁-C₆alkyl, C₁-C₆alkyl-carbonyl, arylcarbonyl, hetarylcarbonyl, arylC₁-C₆alkylearbonyl, hetarylC₁-C₆alkyl-carbonyl, C₁-C₆alkylcarboxy, arylcarboxy or arylC₁-C₆alkylcarboxy;
R¹⁰ and R¹¹ independently are hydrogen, C₁-C₆alkyl, aryl, hetaryl, arylC₁-C₆alkyl, C₃-C₁₀-cycloalkyl, C₃-C₁₀hetcycloalkyl, C₃-C₁₀cycloalkylC₁-C₆alkyl, C₁-C₆alkylcarboxyC₁-C₆alkyl; or
R¹⁰ and R¹¹ together with the nitrogen to which they are attached, are forming a saturated or partially saturated cyclic, bicyclic or tricyclic ring system containing from 4 to 10 carbon atoms and from 0 to 2 additional heteroatoms selected from nitrogen, oxygen or sulphur, the ring system optionally being substituted with at least one of C₁-C₈alkyl, aryl, hetaryl, arylC₁-C₆alkyl, hetarylC₁-C₆alkyl, hydroxy, oxo, cyano, C₁-C₆alkyloxy, arylC₁-C₆alkyloxy, hetaryl C₁-C₆alkyloxy, C₁-C₆alkyloxyC₁-C₆alkyl, C₁-C₆alkylcarbonyl, arylcarbonyl, hetarylcarbonyl, arylC₁-C₆alkylcarbonyl, hetarylC₁-C₆alkylcarbonyl, C₁-C₆alkylcarboxy, arylcarboxy or arylC₁-₆-alkylcarboxy; or
a salt thereof with a pharmaceutically acceptable acid or base, or any optical isomer or mixture of optical isomers, including a racemic mixture, or any tautomeric forms.

3. A compound according to claim 1 or 2 wherein R¹ is aryl, arylC₁-C₆alkyl or hetaryl optionally substituted with one or more of R⁶.

4. A compound according to any of the claims 1-3 wherein R² is C₁-C₆alkyl, C₃-C₁₀cycloalkyl, C₃-C₁₀cycloalkylC₁-C₆alkyl, trihalomethyl, arylC₁-C₆alkyl, or hetarylC₁-C₆alkyl wherein the alkyl, cycloalkyl and aryl groups independently are optionally substituted with one or more R⁷.

5. A compound according to any of the claims 1-4 wherein R³ is C₁-C₆alkyl optionally substituted with one or more of R⁸.

6. A compound according to any of the claims 1-5 wherein R⁴ is C₆-C₁₀cycloalkyl, or C₆-C₁₀hetcycloalkyl, wherein the cycloalkyl and hetcycloalkyl groups independently are optionally substituted with one or more of R⁸.

7. A compound according to any of the claims 1-6 wherein R⁶ and R⁷ independently are hydrogen, hydroxy, oxo, halo, cyano, C₁-C₆alkyl, C₁-C₆alkyloxy, trihalomethyl, NR¹⁰R¹¹, arylC₁-C₆alkyloxy, hetarylC₁-C₆alkyloxy, C₁-C₆alkylcarbonyl, arylcarbonyl, hetarylcarbonyl, arylC₁-C₆alkylcarbonyl, C₁-C₆alkyloxycarbonyl, aryloxycarbonyl or arylC₁-C₆alkyloxycarbonyl.

8. A compound according to any of the claims 1-7 wherein R⁸ and R⁹ independently are hydrogen, C₁-C₆alkyl, hydroxy, oxo, C₁-C₆alkyloxy or arylC₁-C₆alkyloxy.

9. A compound according to any of the claims 1-8 wherein R¹⁰ and R¹¹ independently are hydrogen or C₁-C₈alkyl.

10. A compound according to any of the claims 1-9 which is 1-(4-Chloro-phenyl)-5-propyl-1*H*-pyrazole-4-carboxylic acid cyclohexyl-methyl-amide, or a salt thereof with a pharmaceutically acceptable acid or base, or any optical isomer or mixture of optical isomers, including a racemic mixture, or any tautomeric forms.

11. A compound according to any of the claims 1-9 selected from the group consisting of:
1-(4-Chloro-phenyl)-5-trifluoromethyl-1H-pyrazole-4-carboxylic acid cyclohexyl-methyl-amide;
[1-(4-Methoxy-phenyl)-5-methyl-1*H*-pyrazol-4-yl]-(1,3,3-trimethyl-6-aza-bicyclo[3.2.1]oct-6-yl)-methanone;
1[1-(4-Chloro-phenyl)-5-propyl-1*H*-pyrazol-4-yl]-(1,3,3-trimethyl-6-aza-bicyclo[3.2.1]oct-6-yl)-methanone;
[1-(3,5-Dichloro-phenyl)-5-propyl-1*H*-pyrazol-yl]-(1,3,3-trimethyl--6-aza-bicyclo[3.2.1]oct-6-yl)-methanone; or
a salt thereof with a pharmaceutically acceptable acid or base, or any optical isomer or mixture of optical isomers, including a racemic mixture, or any tautomeric forms.

12. A compound according to any of the claims 1-9 selected from the group consisting of:
1-(Phenyl)-5-methyl-1*H*-pyrazole-4-carboxylic acid cyclohexyl-methyl-amide;
1-(4-Fluoro-phenyl)-5-methyl-1*H*-pyrazole-4-carboxylic acid cyclohexyl-methyl-amide;
1-(4-Methoxy-phenyl)-5-methyl-1*H*-pyrazole-4-carboxylic acid cyclohexyl-methyl-amide;
1-(4-Chloro-phenyl)-5-methyl-1*H*-pyrazole-4-carboxylic acid cyclohexyl-methyl-amide;
1-(2-Methyl-phenyl)-5-methyl-1*H*-pyrazole-4-carboxylic acid cyclohexyl-methyl-amide;
1-(4-Amino-phenyl)-5-methyl-1*H*-pyrazole-4-carboxylic acid cyclohexyl-methyl-amide;
1-(2-Pyridyl)-5-methyl-1*H*-pyrazole-4-carboxylic acid cyclohexyl-methyl-amide;
1-(2-Pyridyl)-5-propyl-1*H*-pyrazole-4-carboxylic acid cyclohexyl-methyl-amide; or
a salt thereof with a pharmaceutically acceptable acid or base, or any optical isomer or mixture of optical isomers, including a racemic mixture, or any tautomeric forms.

13. A combination comprising a compound as defined in any one of claims 1 to 12 and one or more further active substances.

14. The combination according to claim 13 wherein the further active substances are selected from CART (cocaine amphetamine regulated transcript) agonists, NPY (neuropeptide Y) antagonists, MC4 (melano-cortin 4)agonists, orexin antagonists, TNF (tumor necrosis factor) agonists, CRF (corticotropin releasing factor) agonists, CRF BP (corticotropin releasing factor binding protein) antagonists, urocortin agonists, β3 agonists, MSH (melanocyte-stimulating hormone) agonists, MCH (melanocyte-concentrating hormone) antagonists, CCK (cholecystokinin) agonists, serotonin re-uptake inhibitors, serotonin and noradrenaline re-uptake inhibitors, mixed serotonin and noradrenergic compounds, 5HT (serotonin) agonists, bombesin agonists, galanin antagonists, growth hormone, growth hormone releasing compounds, TRH (thyreotropin releasing hormone) agonists, UCP 2 or 3 (uncoupling protein 2 or 3) modulators, leptin agonists, DA agonists (bromocriptin, doprexin), lipase/amylase inhibitors, PPAR (peroxisome proliferator-activated receptor) modulators, RXR (retinoid X receptor) modulators, TR β agonists, AGRP (Agouti related protein) inhibitors, H3 histamine antagonists, opioid antagonists (such as naltrexone), exendin-4, GLP-1 and ciliary neurotrophic factor.

15. A pharmaceutical composition comprising, as an active ingredient, at least one compound according to any one of the claims 1-12 or a combination according to claim 13 or claim 14, together with one or more pharmaceutically acceptable carriers or excipients.

16. The pharmaceutical composition according to claim 15 which is for oral, nasal, buccal, transdermal, pulmonal or parenteral administration.

17. The pharmaceutical composition according to claim 15 or 16 in unit dosage form, comprising from 0.05 mg to 2000 mg/day, from 0.1 mg to 1000 mg or from 0.5 mg to 500 mg per day of the compound according to anyone of the claims 1-12.

18. A compound as defined in any one of claims 1 to 12, or a combination as defined in claim 13 or claim 14, or a composition as defined in any one of claims 15 to 17, for use as a medicament.

19. The compound, combination or composition according to claim 18 for use in the treatment, prevention and/or prophylaxis of any conditions, disorders and diseases wherein a modulation or an inhibition of the activity of 11βHSD1 is beneficial.

20. The compound, combination or composition according to claim 18 for use in the treatment, prevention and/or prophylaxis of any conditions, disorders and diseases that are influenced by intracellular glucocorticoid levels.

21. The compound, combination or composition according to claim 18 for use in the treatment, prevention and/or prophylaxis of conditions, disorders or diseases selected from the group consisting of the metabolic syndrome, insulin resistance, dyslipidemia, hypertension and obesity.

22. The compound, combination or composition according to claim 18 for use in the treatment, prevention and/or prophylaxis of type 2 diabetes, impaired glucose tolerance (IGT), impaired fasting glucose (IFG).

23. The compound, combination or composition according to claim 18 for use in the delaying or prevention of the progression from IGT into type 2 diabetes.

24. The compound, combination or composition according to claim 18 for use in delaying or preventing the progression of the metabolic syndrome into type 2 diabetes.

25. The compound, combination or composition according to claim 18 for use in the treatment, prevention and/or prophylaxis of adverse effects of glucocorticoid receptor agonist treatment or therapy.

26. Use of a compound as defined in any of the claims 1-12, or a combination as defined in claim 13 or 14 for the preparation of a medicament for the treatment, prevention and/or prophylaxis of a condition, disorder or disease as defined in any of claims 19 to 25.

27. A process for preparing a compound as defined in claim 1 said process comprising: 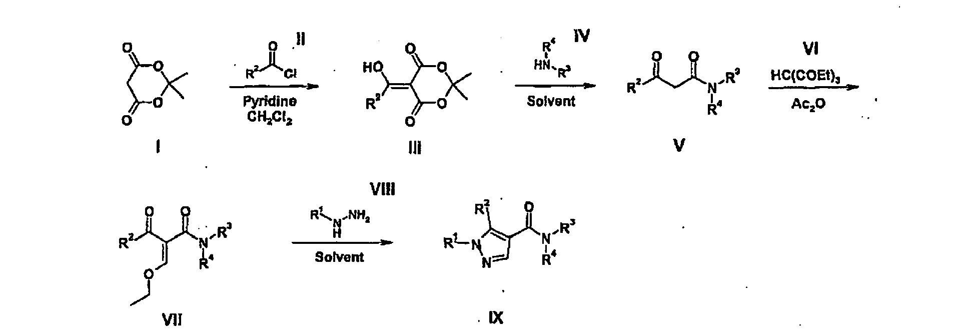 reacting 2,2-dimethyl-1,3-dioxane-4,6-dione (Meldrum's acid) (1) with an acid chloride (II), wherein R² is defined in claim 1, in a solvent mixture of pyridine and DCM. to afford an acyl Meldrum's acid (III), wherein R² is as defined in claim 1 aminolysing the acyl Meldrum's acid (III) with an substituted amine (IV), wherein R³ and R⁴ are as defined in claim 1, in a solvent such as benzene, toluene, dioxane at a temperature interval from 50°C up to reflux to afford a *beta*-keto amide (V) wherein R², R³ and R⁴ are defined in claim 1; treating the *beta*-keto amide (V) with *ortho*-formiate (VI) in a solvent such as acetic acid anhydride at a temperature interval from 50°C up to reflux to afford enol ether (VII) wherein R², R³ and R⁴ are as defined in claim 1; condensing the enol ether (VII) Where R², R³, and R4 are as defined in claim 1 with hydrazide (VIII) wherein R¹ is as defined in claim 1 to yield pyrazol (IX) wherein R¹, R², R³, and R⁴ are as defined in claim 1, in a solvent such as EtOH, iPrOH, tert-BuOH.

## Patentansprüche

1. Verbindung mit der Formel (III): 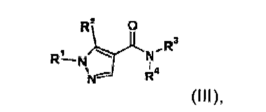 wobei
R¹ Aryl, Aryl-C₁-C₆-alkyl, Hetaryl oder Hetaryl-C₁-C₆-alkyl ist, das optional mit einem oder mehreren R⁶ unabhängig substituiert ist,
R² Halogen, C₁-C₆-Alkyl, C₂-C₆-Alkenyl, C₂-C₆-Alkinyl, C₃-C₁₀-Cyrloalkyl, C₃-C₁₀-Cycloalkyl-C₁-C₆-alkyl, Trihalogenmethyl, Aryl, Aryl-C₁-C₆-alkyl, C₁-C₆-Alkyloxy-C₁-C₆-alkyl, Aryl-C₁-C₆-alkyloxy-C₁-C₆-alkyl, C₁-C₆-Alkyl-NR⁵-C₁-C₆-alkyl, Aryl-C₁-C₆-alkyl-NR⁵-C₁-C₆-alkyl, Hetaryl oder Hetaryl-C₁-C₆-alkyl ist, wobei die Alkyl-, Alkenyl-, Akinyl-, Cycloalkyl- und Arylgruppen unabhängig optional mit einem oder mehreren R⁷ substituiert sind,
R³ C₁-C₆-Alkyl ist, das optional mit einem oder mehreren R⁸ substituiert ist,
R⁴ C₆-C₁₀-Cycloalkyl, C₆-C₁₀-Hetcycloalkyl, C₆-C₁₀-Cycloalkyl-C₁-C₆-alkyl oder C₆-C₁₀-Hetcycloalkyl-C₁-C₆-alkyl ist, wobei die Alkyl-, Cycloalkyl- und Hetcycloalkylgruppen unabhängig optional mit einem oder mehreren R⁸ substituiert sind, oder
R³ und R⁴ zusammen mit dem Stickstoff, mit dem sie verknüpft sind, ein gesättigtes oder teilweise gesättigtes bicyclisches/verbrücktes Ringsystem bilden, das 7 bis 12 Kohlenstoffatome und 0 bis 2 zusätzliche Heteroatome ausgewählt aus Stickstoff, Sauerstoff oder Schwefel enthält, wobei das Ringsystem optional mit C₁-C₆-Alkyl, Aryl, Hetaryl, Aryl-C₁-C₆-alkyl, Hetaryl-C₁-C₆-alkyl, Hydroxy, Oxo, C₁-C₆-Alkyloxy, Aryl-C₁-C₆-alkyloxy und/oder Hetaryl-C₁-C₆-alkyloxy substituiert ist, wobei die Alkyl- und Arylgruppen unabhängig optional mit einem oder mehreren R⁹ substituiert sind,
R⁵ C₁-C₆-Alkyl, C₂-C₆-Alkenyl, C₂-C₆-Alkinyl, C3-C₁₀-Cycloalkyl, C₃-C₁₀-Hetcyclcalkyl, C₃-C₁₀-Cycloalkyl-C₁-C₆-alkyl, C₃-C₁₀-Hetcycloalkyl-C₁-C₆-alkyl, Aryl, Hetaryl, Aryl-C₁-C₆-alkyl oder Hetaryl-C₁-C₆-alkyl ist, wobei die Alkyl-, Alkenyl-, Akinyl-, Aryl-, Hetaryl, Cycloalkyl- und Hetcycloalkylgruppen unabhängig optional mit einem oder mehreren R⁹ substituiert sind,
R⁵ und R⁷ unabhängig Wasserstoff, Hydroxy, Oxo, Halogen, Nitro, Cyano, C₁-C₆-Alkyl, C₁-C₆-Alkyloxy, Trihalogenmethyl, Trihalogenmethoxy, NR¹⁰R¹¹, Aryl-C₁-C₆-alkyloxy, Hetaryl-C₁-C₆-alkyloxy, C₁-C₆-Alkylcarbonyl, Arylcarbonyl, Hetarylcarbonyl, Aryl-C₁-C₆-alkylcarbonyl, C₁-C₆-Alkyloxycarbonyl, Aryloxycarbonyl, Aryl-C₁-C₆-alkyloxycarbonyl, C₁-C₆-Alkylcarboxy, Arylcarboxy oder Aryl-C₁-C₆-alkylcarboxy sind,
R⁸ und R⁹ unabhängig Wasserstoff, C₁-C₆-Alkyl, Aryl, Hetaryl, Aryl-C₁-C₈-alkyl, Hetaryl-C₁-C₆-alkyl, Hydroxy, Oxo, Cyano, NR¹⁰R¹¹, C₁-C₆-Alkyloxy, Aryloxy, Aryl-C₁-C₆-alkyloxy, Hetaryloxy, Hetaryl-C₁-C₆-alkyloxy, C₁-C₆-Alkyloxy-C₁-C₆-alkyl, C₁-C₆-Alkylcarbonyl, Arylcarbonyl, Hetarylcarbonyl, Aryl-C₁-C₆-alkylcarbonyl, Hetaryl-C₁-C₆-alkylcarbonyl, C₁-C₆-Alkylcarboxy, Arylcarboxy oder Aryl-C₁-C₆-Alkylcarboxy sind,
R¹⁰ und R¹¹ unabhängig Wasserstoff, C₁-C₈-Alkyl, Aryl, Hetaryl, Aryl-C₁-C₆-alkyl, C₃-C₁₀-Cycloalkyl. C₃-C₁₀-Hetcycloalkyl, C₃-C₁₀-Cycloalkyl-C₁-C₆-alkyl, C₁-C₆-Alkylcarbonyl, C₁-C₆-Alkylcarboxy-C₁-C₆-alkyl sind, oder R¹⁰ und R¹¹ zusammen mit dem Stickstoff, mit dem sie verknüpft sind, ein gesättigtes oder teilweise gesättigtes cyclisches, bicyclisches oder tricyclisches Ringsystem bilden, das 4 bis 10 Kohlenstoffatome und 0 bis 2 zusätzliche Heteroatome ausgewählt aus Stickstoff, Sauerstoff oder Schwefel enthält, wobei das Ringsystem optional mit C₁-C₈-Alkyl, Aryl, Hetaryl, Aryl-C₁-C₆-alkyl, Fietaryl-C₁-C₆-alkyl, Hydroxy, Oxo, Cyano, C₁-C₆-Alkyloxy, Aryl-C₁-C₆-alkyloxy, Hetaryl-C₁-C₆-alkyloXy, C₁-C₆-AlKyloxy-C₁-C₆-alkyl, C₁-C₆-Alkylcarbonyl, Arylcarbonyl, Hetarylcarbonyl, Aryl-C₁-C₆-alkylcarbonyl, Hetaryl-C₁-C₆-alkylcarbonyl, C₁-C₆-Alkylcarboxy, Arylcarboxy und/oder Aryl-C₁-C₆-alkylcarboxy substituiert ist, oder
ein Salz davon mit einer pharmazeutisch akzeptablen Säure oder Base oder beliebige optische Isomere oder Mischungen von optischen Isomeren, darunter eine racemische Mischung, oder beliebige tautomere Formen.

2. Verbindung nach Anspruch 1, wobei R¹ Aryl oder Hetaryl ist, das optional mit einem oder mehreren R⁶ unabhängig substituiert ist,
R² Halogen, C₁-C₆-Alkyl, C₂-C₆-Alkenyl, C₂-C₆-Alkinyl, C₃-C₁₀-Cycloalkyl, C₃-C₁₀-Cycloalkyl-C₁-C₆-alkyl, Aryl, Aryl-C₁-C₆-alkyl, C₁-C₆-Alkyloxy-C₁-C₆-alkyl, Aryl-C₁-C₆-alkyloxy-C₁-C₆-alkyl, C₁-C₆-Alkyl-NR⁵-C₁-C₆-alkyl, Aryl-C₁-C₆-alkyl-NR⁵-C₁-C₆-alkyl, Hetaryl oder Hetaryl-C₁-C₆-alkyl ist, wobei die Alkyl-, Alkenyl-, Akinyl-, Cycloalkyl- und Arylgruppen unabhängig optional mit einem oder mehreren R⁷ substituiert sind,
R³ C₁-C₆-Alkyl ist, das optional mit einem oder mehreren R⁸ substituiert ist,
R⁴ C₆-C₁₀-Cycloalkyl, C₆-C₁₀-Hetcycloalkyl, C₆-C₁₀-Cycloalkyl-C₁-C₆-alkyl oder C₆-C₁₀-Hetcycloalkyl-C₁-C₆-alkyl ist, wobei die Alkyl-, Cycloalkyl- und Hetcycloalkylgruppen unabhängig optional mit einem oder mehreren R⁸ substituiert sind,
R⁵ C₁-C₆-Alkyl, C₂-C₆-Alkenyl, C₂-C₆-Alkinyl, C₃-C₁₀-Cycloalkyl, C₃-C₁₀-Hetcycloalkyl, C₃-C₁₀-Cycloalkyl-C₁-C₆-alkyl, C₃-C₁₀-Hetcycloalkyl-C₁-C₆-alkyl, Aryl, Hetaryl, Aryl-C₁-C₆-alkyl oder Hetaryl-C₁-C₆-alkyl ist, wobei die Alkyl-, Alkenyl-, Akinyl-, Aryl-, Hetaryl-, Cycloalkyl- und Hetcycloalkylgruppen unabhängig optional mit einem oder mehreren R⁹ substituiert sind,
R⁶ und R⁷ unabhängig Wasserstoff, Hydroxy, Oxo, Halogen, Nitro, Cyano, C₁-C₆-Alkyl, C₁-C₆-Alkyloxy, Trihalogenmethyl, Trihalogenmethoxy, NR¹⁰R¹¹, Aryl-C₁-C₆-alkyloxy, Hetaryl-C₁-C₆-alkyloxy, C₁-C₆-Alkylcarbonyl, Arylcarbonyl, Hetarylcarbonyl, Aryl-C₁-C₆-alkylcarbonyl, C₁-C₆-Alkyloxycarbanyl, Aryloxycarbonyl, Aryl-C₁-C₆-alkyloxycalrbonyl, C₁-C₆-Alkylcarboxy, Arylcarboxy oder Aryl-C₁-C₆₋alkylcarboxy sind,
R⁸ und R⁹ unabhängig Wasserstoff, C₁-C₆-Alkyl, Aryl, Hetaryl, Aryl-C₁-C₆-Alkyl, Hetaryl-C₁-C₆-Alkyl, Hydroxy, Oxo, Cyano, NR¹⁰R¹¹, C₁-C₆-Alkyloxy, Aryloxy, Aryl-C₁-C₆-alkyloxy, Hetaryloxy, Hetaryl-C₁-C₆-alkyloxy, C₁-C₆-Alkyloxy-C₁-C₆-alkyl, C₁-C₆-Alkylcarbonyl, Arylcarbonyl, Hetarylcarbonyl, Aryl-C₁-C₆-alkylcarbonyl, Hetaryl-C₁-C₆-alkyloarbonyl, C₁-C₆-Alkylcarboxy, Arylcarboxy oder Aryl-C₁-C₆-alkylcarboxy sind,
R¹⁰ und R¹¹ unabhängig Wasserstoff, C₁-C₈-Alkyl, Aryl, Hetaryl, Aryl-C₁-C₆-alkyl, C₃-C₁₀-Cycloalkyl, C₃-C₁₀-Hetcycloalkyl, C₃-C₁₀-Cycloalkyl-C₁-C₆-alkyl, C₁-C₆-Alkylcarboxy-C₁-C₆-alkyl sind, oder
R¹⁰ und R¹¹ zusammen mit dem Stickstoff, mit dem sie verknüpf: sind, ein gesättigtes oder teilweise gesättigtes cyclisches, bicyclisches oder tricyclisches Ringsystem bilden, das 4 bis 10 Kohlenstoffatome und 0 bis 2 zusätzliche Heteroatome ausgewählt aus Stickstoff, Sauerstoff oder Schwefel enthält, wobei das Ringsystem optional mit C₁-C₈-Alkyl, Aryl, Hetaryl, Aryl-C₁-C₆-Alkyl, Hetaryl-C₁-C₆-alkyl, Hydroxy, Oxo, Cyano, C₁-C₆-Alkyloxy, Aryl-C₁-C₆-alkyloxy, Hetaryl-C₁-C₆-alkyloxy, C₁-C₆-Alkyloxy-C₁-C₆-alkyl, C₁-C₆-Alkylcarbonyl, Arylcarbonyl, Hetarylcarbonyl, Aryl-C₁-C₆-alkylcarbonyl, Hetaryl-C₁-C₆-alkylcarbonyl, C₁-C₆-Alkylcarboxy, Arylcarboxy und/oder Aryl-C₁-C₆-alkylcarboxy substituiert ist, oder
ein Salz davon mit einer pharmazeutisch akzeptablen Säure oder Base oder beliebige optische Isomere oder Mischungen von optischen Isomeren, darunter eine racemische Mischung, oder beliebige tautomere Formen.

3. Verbindung nach Anspruch 1 oder 2, wobei R¹ Aryl, Aryl-C₁-C₆-Alkyl oder Hetaryl ist, das optional mit einem oder mehreren R⁶ substituiert ist.

4. Verbindung nach einem der Ansprüche 1 bis 3, wobei R² C₁-C₆-Alkyl, C₃-C₁₀-Cycloalkyl, C₃-C₁₀-Cycloalkyl-C₁-C₆-alkyl, Trihalogenmethyl, Aryl-C₁-C₆-alkyl oder Hetaryl-C₁-C₆-alkyl ist, wobei die Alkyl-, Cycloalkyl- und Arylgruppen unabhängig optional mit einem oder mehreren R⁷ substituiert sind.

5. Verbindung nach einem der Ansprüche 1 bis 4, wobei R³ C₁-C₆-Alkyl ist, das optional mit einem oder mehreren R⁸ substituiert ist.

6. Verbindung nach einem der Ansprüche 1 bis 5, wobei R⁴ C₆-C₁₀-Cycloalkyl oder C₆-C₁₀-Hetcycloalkyl ist, wobei die Cycloalkyl- und Hetcycloalkylgruppen unabhängig optional mit einem oder mehreren R⁸ substituiert sind.

7. Verbindung nach einem der Ansprüche 1 bis 6, wobei R⁶ und R⁷ unabhängig Wasserstoff, Hydroxy, Oxo, Halogen, Cyano, C₁-C₆-Alkyl, C₁-C₆-Alkyloxy, Trihalogenmethyl, NR¹⁰R¹¹, Aryl-C₁-C₆-alkyloxy, Hetaryl-C₁-C₆-alkyloxy, C₁-C₆-Alkyloarbonyl, Arylcarbonyl, Hetarylcarbonyl, Aryl-C₁-C₆alkylcarbonyl, C₁-C₆-Alkyloxycarbonyl, Aryloxycarbonyl order Aryl-C₁-C₆-alkyloxycarbonyl sind.

8. Verbindung nach einem der Ansprüche 1 bis 7, wobei R⁸ und R⁹ unabhängig Wasserstoff, C₁-C₆-Alkyl, Hydroxy, Oxo, C₁-C₆-Alkyloxy oder Aryl-C₁-C₆-alkyloxy sind.

9. Verbindung nach einem der Ansprüche 1 bis 8, wobei R¹⁰ und R¹¹ unabhängig Wasserstoff oder C₁-C₈-Alkyl sind.

10. Verbindung nach einem der Ansprüche 1 bis 9, die 1-(4-Chlorphenyl)-5-propyl-1*H*-pyrazol-4-carbonsäure-cyclohexyl-methyl-amid oder ein Salz davon mit einer pharmazeutisch akzeptablen Säure oder Base oder beliebige optische Isomere oder Mischungen von optischen Isomeren, darunter eine racemische Mischung, oder beliebige tautomere Formen darstellt.

11. Verbindung nach einem der Ansprüche 1 bis 9, ausgewählt aus der Gruppe bestehend aus:
1-(4-Chlor-phenyl)-5-trifluormethyl-1*H*-pyrazol-4-carbonsäure-cyclohexyl-methyl-amid,
[1-(4-Methoxy-phenyl)-5-methyl-1*H*-pyrazol-4-yl]-(1,3,3-trimethyl-6-azabicyclo[3.2.1)oct-6-yl)-methanon,
1-[1-(4-Chlor-phenyl)-5-propyl-1*H*-pyrazol-4-yl]-(1,3,3-trimethyl-6-azabicyclo[3.2.1)oct-6-yl)-methanon,
[1-(3,5-Dichlor-phenyl)-5-propyl-1*H*-pyrazol-4-yl]-(1,3,3-trimethyl-6-azabicyclo[3.2.1)oct-6-yl)-methanon oder
ein Salz davon mit einer pharmazeutisch akzeptablen Säure oder Base oder beliebige optische Isomere oder Mischungen von optischen Isomeren, darunter eine racemische Mischung, oder beliebige tautomere Formen.

12. Verbindung nach einem der Ansprüche 1 bis 9, ausgewählt aus der Gruppe bestehend aus:
1-(Phenyl)-5-methyl-1*H*-pyrazol-4-carbonsäure-cyclohexyl-methyl-amid,
1-(4-Fluor-phenyl)-5-methyl-1*H*-pyrazol-4-carbonsäute-cyclohexyl-methyl-amid,
1-(4-Methoxy-phenyl)-5-methyl-1*H*-pyrazol-4-carbonsäure-cyclohexyl-methyl-amid,
1-(4-Chlor-phenyl)-5-methyl-1*H*-pyrazol-4-carbonsäure-cyclohexyl-methyl-amid,
1-(2-Methyl-phenyl)-5-methyl-1*H*-pyrazol-4-carbonsäure-cyclohexyl-methyl-amid,
1-(4-Amino-phenyl)-5-methyl-1*H*-pyrazol-4-carbonsäure-cyclohexyl-methyl-amid,
1-(2-Pyridyl)-5-methyl-1*H*-pyrazol-4-carbonsäure-cyclohexyl-methylamid,
1-(2-Pyridyl)-5-propyl-1*H*-pyrazol-4-carbonsäure-cyclohexyl-methyl-amid oder
ein Salz davon mit einer pharmazeutisch akzeptablen Säure oder Base oder beliebige optische Isomere oder Mischungen von optischen Isomeren, darunter eine racemische Mischung, oder beliebige tautomere Formen.

13. Kombination umfassend eine Verbindung wie in einem der Ansprüche 1 bis 12 definiert und eine oder mehrere weitere aktive Substanzen.

14. Kombination nach Anspruch 13, wobei die weiteren aktiven Substanzen ausgewählt sind aus CART(Cocain Amphetamine Regulated Transcript)-Agonisten, NPY(Neuropeptid Y)-Antagonisten, MC4(Melanocortin-4)-Agonisten, Orexin-Antagonisten, TNF(Tumor-Nekrose-Faktor)-Agonisten, CRF(Corticotropin Releasing Factor)-Agonisten, CRF BP(Corticotropin Releasing Factor Binding Protein)-Antagonisten, Urocortin-Agonisten, β3-Agonisten, MSH(Melanocyten-Stimulierendes Hormon)-Agonisten, MCH(Melanocyten-konzentrierendes Hormon)-Antagonisten, CCK(Cholecystokinin)-Agonisten, Serotonin-Wiederaufnahme-Inhibitoren, Serotonin- und Noradrenalin-Wiederaufnahme-Inhibitoren, gemischte Serotonin- und noradrenerge Verbindungen, 5HT(Serotonin)-Agonisten, Bombesin-Agonisten, Galanin-Antagonisten, Wachstumshormone, Wachstumshormon freisetzende Verbindungen, TRH(Thyreotropin Releasing Hormone)-Agonisten, UCP2- oder UCP3(Uncoupling Protein 2 oder 3)-Modulatoren, Leptin-Agonisten, DA-Agonisten (Bromocriptin, Doprexin), Lipase/Amylase-Inhibitoren, PPAR(Peroxisom Proliferator-Aktivierte Rezeptor)-Modulatoren, RXR(Retinoid-X-Rezeptor)-Modulatoren, TR β-Agonisten, AGRP(Agouti Related Protein)-Inhibitoren, H3-Histamin-Antagonisten, Opioid-Antagonisten (wie Naltrexon), Exendin-4, GLP-1 und CNTF(Ciliärer Neurotropher Faktor).

15. Pharmazeutische Zusammensetzung umfassend, als aktiven Wirkstoff, mindestens eine Verbindung nach einem der Ansprüche 1 bis 12 oder eine Kombination nach Anspruch 13 oder Anspruch 14, zusammen mit einem oder mehreren pharmazeutisch akzeptablen Trägern oder Exzipienten.

16. Pharmazeutische Zusammensetzung nach Anspruch 15, die für orale, nasale, buccale, transdermale, pulmonale oder parenterale Verabreichung vorgesehen ist.

17. Pharmazeutische Zusammensetzung nach Anspruch 15 oder 16, in Form von Dosiseinheiten, umfassend von 0,05 mg bis 2000 mg/Tag, von 0,1 mg bis 1000 mg oder von 0,5 mg bis 500 mg pro Tag der Verbindung nach einem der Ansprüche 1 bis 12.

18. Verbindung wie in einem der Ansprüche 1 bis 12 definiert oder eine Kombination wie in Anspruch 13 oder Anspruch 14 definiert oder eine Zusammensetzung wie in einem der Ansprüche 15 bis 17 definiert, zur Verwendung als Medikament.

19. Verbindung, Kombination oder Zusammensetzung nach Anspruch 18, zur Verwendung bei der Behandlung, Vorbeugung und/oder Prophylaxe beliebiger Zustände, Störungen und Erkrankungen, bei denen eine Modulation oder eine Hemmung der Aktivität von 11βHSD1 vorteilhaft ist.

20. Verbindung, Kombination oder Zusammensetzung nach Anspruch 18, zur Verwendung bei der Behandlung, Vorbeugung und/oder Prophylaxe beliebiger Zustände, Störungen und Erkrankungen, die durch intrazelluläre Glucocorticoidspiegel beeinflusst sind.

21. Verbindung, Kombination oder Zusammensetzung nach Anspruch 18, zur Verwendung bei der Behandlung, Vorbeugung und/oder Prophylaxe von Zuständen, Störungen oder Erkrankungen, die ausgewählt sind aus der Gruppe bestehend aus dem metabolischen Syndrom, Insulinresistenz, Dyslipidämie, Bluthochdruck (Hypertension) und Fettleibigkeit (Obesität).

22. Verbindung, Kombination oder Zusammensetzung nach Anspruch 18, zur Verwendung bei der Behandlung, Vorbeugung und/oder Prophylaxe von Typ-2-Diabetes, verminderter Glucosetoleranz (IGT), abnormer Nüchternglucose (IFG).

23. Verbindung, Kombination oder Zusammensetzung nach Anspruch 18, zur Verwendung bei der Verzögerung oder Verhinderung des Fortschreitens einer IGT zu einem Typ-2-Diabetes.

24. Verbindung, Kombination oder Zusammensetzung nach Anspruch 18, zur Verwendung bei der Verzögerung oder Verhinderung des Fortschreitens eines metabolischen Syndroms zu einem Typ-2-Diabetes.

25. Verbindung, Kombination oder Zusammensetzung nach Anspruch 18, zur Verwendung bei der Behandlung, Vorbeugung und/oder Prophylaxe von Beeinträchtigungen einer Glucocorticoidrezeptoragonisten-Behandlung oder -Therapie.

26. Verwendung einer Verbindung wie in einem der Ansprüche 1 bis 12 definiert oder einer Kombination wie in Anspruch 13 oder 14 definiert zur Herstellung eines Medikaments zur Behandlung, Vorbeugung und/oder Prophylaxe eines Zustands, einer Störung oder einer Erkrankung wie in einem der Ansprüche 19 bis 25 definiert.

27. Verfahren zur Herstellung einer Verbindung wie in Anspruch 1 definiert, wobei das Verfahren umfasst: 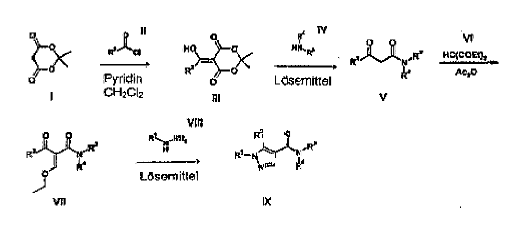 Umsetzen von 2,2-Dimethyl-1,3-dioxan-4,6-dion (Meldrumsäure) (I) mit einem Säurechlorid (II), wobei R² wie in Anspruch 1 definiert ist, in einem Lösungsmittelgemisch aus Pyridin und DCM, um eine Acylmeldrumsäure (III) zu bilden, wobei R² wie in Anspruch 1 definiert ist; Aminolysieren der Acylmeldrumsäure (III) mit einem substituierten Amin (IV), wobei R³ und R⁴ wie in Anspruch 1 definiert sind, in einem Lösungsmittel wie Benzol, Toluol, Dioxan in einem Temperaturintervall von 50 °C bis zum Rückfluss, um ein *beta*-Ketoamid (V) zu bilden, wobei R², R³ und R⁴ wie in Anspruch 1 definiert sind; Behandeln des *beta*-Ketoamids (V) mit *ortho*-Formiat (VI) in einem Lösungsmittel wie Essigsäureanhydrid in einem Temperaturintervall von 50 °C bis zum Rückfluss, um Enolether (VII) zu bilden, wobei R², R³ und R⁴ wie in Anspruch 1 definiert sind; Kondensieren des Enolethers (VII), wobei R², R³ und R⁴ wie in Anspruch 1 definiert sind, mit Hydrazid (VIII), wobei R¹ wie in Anspruch 1 definiert ist, so das Pyrazol (IX) erhalten wird, wobei R¹, R², R³ und R⁴ wie in Anspruch 1 definiert sind, in einem Lösungsmittel wie EtOH, iPrOH, tert-BuOH.

## Revendications

1. Composé de formule (III) : 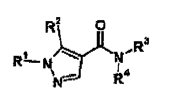 dans laquelle
R¹ est un groupe aryle, aryl-alkyle en C₁ à C₆, hétaryle ou hétaryl-alkyle en C₁ à C₆ facultativement substitué par un ou plusieurs de R⁶ indépendamment ;
R² est un groupe halogéno, alkyle en C₁ à C₆, alcényle en C₂ à C₆, alcynyle en C₂ à C₆, cycloalkyle en C₃ à C₁₀, cycloalkyl en C₃ à C₁₀-alkyle en C₁ à C₆, trihalogénométhyle, aryle, aryl-alkyle en C₁ à C₆, alkyloxy en C₁ à C₆-alkyle en C₁ à C₆, aryl-alkyloxy en C₁ à C₆-alkyle en C₁ à C₆, alkyl en C₁ à C₆-NR⁵-alkyle en C₁ à C₆, aryl-alkyl en C₁ à C₆-NR⁵-alkyle en C₁ à C₆, hétaryle ou hétaryl-alkyle en C₁ à C₆ où les groupes alkyle, alcényle, alcynyle, cycloalkyle et aryle sont indépendamment facultativement substitués par un ou plusieurs R⁷ ;
R³ est un groupe alkyle en C₁ à C₆ facultativement substitué par un ou plusieurs de R⁸ ;
R⁴ est un groupe cycloalkyle en C₆ à C₁₀, hetcycloalkyle en C₆ à C₁₀, cycloalkyl en C₆ à C₁₀-alkyle en C₁ à C₆ ou hetcycloalkyl en C₆ à C₁₀-alkyle en C₁ à C₆, où les groupes alkyle, cycloalkyle et hetcycloalkyle sont indépendamment facultativement substitués par un ou plusieurs de R⁸ ; ou
R³ et R⁴ conjointement avec l'atome d'azote auquel ils sont attachés, forment un système de cycle bicyclique/de pont saturé ou partiellement saturé contenant 7 à 12 atomes de carbone et 0 à 2 hétéroatomes additionnels choisis parmi l'azote, l'oxygène ou le soufre, le système de cycle étant facultativement substitué par au moins l'un d'un groupe alkyle en C₁ à C₆, aryle, hétaryle, aryl-alkyle en C₁ à C₆, hétaryl-alkyle en C₁ à C₆, hydroxy, oxo, alkyloxy en C₁ à C₆, aryl-alkyloxy en C₁ à C₆ ou hétaryl-alkyloxy en C₁ à C₆, où les groupes alkyle et aryle sont indépendamment facultativement substitués par un ou plusieurs de R⁹ ;
R⁵ est un groupe alkyle en C₁ à C₆, alcényle en C₂ à C₆, alcynyle en C₂ à C₆, cycloalkyl en C₃ à C₁₀, hetcycloalkyle en C₃ à C₁₀, cycloalkyl en C₃ à C₁₀-alkyle en C₁ à C₆, hetcycloalkyl en C₃ à C₁₀-alkyle en C₁ à C₆, aryle, hétaryle, aryl-alkyle en C₁ à C₆ ou hétaryl-alkyle en C₁ à C₆, où les groupes alkyle, alcényle, alcynyle, aryle, hétaryle, cycloalkyle et hetcycloalkyle sont indépendamment facultativement substitués par un ou plusieurs de R⁹ ;
R⁶ et R⁷ sont indépendamment un atome d'hydrogène, un groupe hydroxy, oxo, halogéno, nitro, cyano, alkyle en C₁ à C₆, alkyloxy en C₁ à C₆, trihalogénométhyle, trihalogénométhoxy, NR¹⁰R¹¹, aryl-alkyloxy en C₁ à C₆, hétaryl-alkyloxy en C₁ à C₆, alkylcarbonyle en C₁ à C₆, arylcarbonyl, hétarylcarbonyle, aryl-alkylcarbonyle en C₁ à C₆, alkyloxycarbonyle en C₁ à C₆, aryloxycarbonyle, aryl-alkyloxycarbonyle en C₁ à C₆, alkylcarboxy en C₁ à C₆, arylcarboxy ou aryl-alkyle en C₁ à C₆-carboxy ;
R⁸ et R⁹ sont indépendamment un atome d'hydrogène, un groupe alkyle en C₁ à C₆, aryle, hétaryle, aryl-alkyle en C₁ à C₆, hétaryl-alkyle en C₁ à C₆, hydroxy, oxo, cyano, NR¹⁰R¹¹, alkyloxy en C₁ à C₆, aryloxy, arylalkyloxy en C₁ à C₆, hétaryloxy, hétaryl-alkyloxy en C₁ à C₆, alkyloxy en C₁ à C₆-alkyle en C₁ à C₆, alkylcarbonyle en C₁ à C₆, arylcarbonyle, hétarylcarbonyle, aryl-alkylcarbonyle en C₁ à C₆, hétaryl-alkylcarbonyle en C₁ à C₆, alkylcarboxy en C₁ à C₆, arylcarboxy ou aryl-alkylcarboxy en C₁ à C₆;
R¹⁰ et R¹¹ sont indépendamment un atome d'hydrogène, un groupe alkyle en C₁ à C₈, aryle, hétaryle, aryl-alkyle en C₁ à C₆, cycloalkyle en C₃ à C₁₀, hetcycloalkyle en C₃ à C₁₀, cycloalkyl en C₃ à C₁₀-alkyle en C₁ à C₆, alkylcarbonyle en C₁ à C₆, alkyl en C₁ à C₆-carboxy-alkyle en C₁ à C₆ ; ou
R¹⁰ et R¹¹ conjointement avec l'atome d'azote auquel ils sont attachés, forment un système de cycle cyclique, bicyclique ou tricyclique saturé ou partiellement saturé contenant 4 à 10 atomes de carbone et 0 à 2 hétéroatomes additionnels choisis parmi l'azote, l'oxygène ou le soufre, le système de cycle étant facultativement substitué par au moins l'un d'un groupe alkyle en C₁ à C₈, aryle, hétaryle, aryl-alkyle en C₁ à C₆, hétaryl-alkyle en C₁ à C₆, hydroxy, oxo, cyano, alkyloxy en C₁ à C₆, aryl-alkyloxy en C₁ à C₆, hetaryl-alkyloxy en C₁ à C₆, alkyloxy en C₁ à C₆-alkyle en C₁ à C₆, alkylcarbonyle en C₁ à C₆, arylcarbonyle, hétarylcarbonyle, aryl-alkylcarbonyle en C₁ à C₆, hétaryl-alkylcarbonyle en C₁ à C₆, alkylcarboxy en C₁ à C₆, arylcarboxy ou aryl-alkylcarboxy en C₁ à C₆ ; ou
sel de celui-ci avec un acide ou une base pharmaceutiquement acceptable, ou tout isomère optique ou mélange d'isomères optiques, incluant un mélange racémique, ou toute forme tautomère.

2. Composé selon la revendication 1, dans lequel R¹ est un groupe aryle ou hétaryle facultativement substitué par un ou plusieurs R⁶ indépendamment ;
R² est un groupe halogéno, alkyle en C₁ à C₆, alcényle en C₂ à C₆, alcynyle en C₂ à C₆, cycloalkyle en C₃ à C₁₀, cycloalkyl en C₃ à C₁₀-alkyle en C₁ à C₆, aryle, aryl-alkyle en C₁ à C₆, alkyloxy en C₁ à C₆-allyle en C₁ à C₆, aryl-alkyloxy en C₁ à C₆-alkyle en C₁ à C₆ alkyl en C₁ à C₆-NR⁵-alkyle en C₁ à à C₆, aryl-alkyl en C₁ à C₆-NR⁵-alkyle en C₁ à C₆, hétaryle ou hétaryl-alkyle en C₁ à C₆ où les groupes alkyle, alcényle, alcynyle, cycloalkyle et aryle sont indépendamment facultativement substitués par un ou plusieurs R⁷ ;
R³ est un groupe alkyle en C₁ à C₆ facultativement substitué par un ou plusieurs de R⁸ ;
R⁴ est un groupe cycloalkyle en C₆ à C₁₀, hetcycloalkyle en C₆ à C₁₀, cycloalkyl en C₆ à C₁₀-alkyle en C₁ à C₆ ou hetcycloalkyl en C₆ à C₁₀-alkyle en C₁ à C₆, où les groupes alkyle, cycloalkyle et hetcycloalkyle sont indépendamment facultativement substitués par un ou plusieurs de R⁸ ;
R⁵ est un groupe alkyle en C₁ à C₆, alcényle en C₂ à C₆, alcyhyle en C₂ à C₆, cycloalkyle en C₃ à C₁₀, hetcycloalkyle en C₃ à C₁₀, cycloalkyl en C₃ à C₁₀-alkyle en C₁ à C₆, hetcycloalkyl en C₃ à C₁₀-alkyle en C₁ à C₆, aryle, hétaryle, aryl-alkyle en C₁ à C₆ ou hétaryl-alkyle en C₁ à C₆, où les groupes alkyle, alcényle, alcynyle, aryle, hétaryle, cycloalkyl et hetcycloalkyle sont indépendamment facultativement substitués par un ou plusieurs de R⁹ ;
R⁶ et R⁷ sont indépendamment un atome d'hydrogène, un groupe hydroxy, oxo, halogéno, nitro, cyano, alkyle en C₁ à C₆, alkyloxy en C₁ à C₆, trihalogenométhyle, trihalogénométhoxy, NR¹⁰R¹¹, aryl-alkyloxy en C₁ à C₆, hétaryl-alkyloxy en C₁ à C₆, alkylcarbonyle en C₁ à C₆, arylcarbonyle, hétarylcarbonyle, aryl-alkylcarbonyle en C₁ à C₆, alkyloxycarbonyle en C₁ à C₆, aryloxycarbonyle, aryl-alkyloxycarbonyle en C₁ à C₆, alkylcarboxy en C₁ à C₆, arylcarboxy ou aryl-alkyle en C₁ à C₆-carboxy ;
R⁸ et R⁹ sont indépendamment un atome d'hydrogène, un groupe alkyle en C₁ à C₆, aryle, hétaryle, aryl-alkyle en C₁ à C₆, hétaryl-alkyle en C₁ à C₆, hydroxy, oxo, cyano, NR¹⁰R¹¹, alkyloxy en C₁ à C₆, alyloxy, arylalkyloxy en C₁ à C₆, hétaryloxy, hetaryl-alkyloxy en C₁ à C₆, alkyloxy en C₁ à C₆-alkyle en C₁ à C₆, alkylcarbonyle en C₁ à C₆, arylcarbonyl, hétarylcarbonyle, aryl-alkylcarbonyle en C₁ à C₆, hétaryl-allkylcarbonyle en C₁ à C₆, alkylcarboxy en C₁ à C₆, arylcarboxy ou aryl-alkylcarboxy en C₁ à C₆ ;
R¹⁰ et R¹¹ sont indépendamment un atome d'hydrogène, un groupe alkyle en C₁ à C₈, aryle, hétaryle, aryl-alkyle en C₁ à C₆, cycloalkyle en C₃ à C₁₀, hetcycloalkyle en C₃ à C₁₀, cycloalkyl en C₃ à C₁₀-alkyle en C₁ à C₆, alkyl en C₁ à C₆-carboxy-alkyle en C₁ à C₆ ; ou
R¹⁰ et R¹¹ conjointement avec l'atome d'azote auquel ils sont attachés, forment un système de cycle cyclique, bicyclique ou tricyclique saturé ou partiellement saturé contenant 4 à 10 atomes de carbone et 0 à 2 heteroatomes additionnels choisis parmi l'azote, l'oxygène ou le soufre, le système de cycle étant facultativement substitué par au moins l'un d'un groupe alkyle en C₁ à C₈, aryle, hétaryle, aryl-alkyle en C₁ à C₆, hétaryl-alkyle en C₁ à C₆, hydroxy, oxo, cyano, alkyloxy en C₁ à C₆, aryl-alkyloxy en C₁ à C₆, hétaryl-alkyloxy en C₁ à C₆, alkyloxy en C₁ à C₆-alkyle en C₁ à C₆, alkylcarbonyle en C₁ à C₆, arylcarbonyle, hétarylcarbonyle, aryl-alkylcarbonyle en C₁ à C₆, hétaryl-alkylcarbonyle en C₁ à C₆, alkylcarboxy en C₁ à C₆, arylcarboxy ou aryt-alkylcarboxy en C₁ à C₆ ; ou
sel de celui-ci avec un acide ou une base pharmaceutiquement acceptable, ou tout isomère optique ou mélange d'isomères optiques, incluant un mélange racémique, ou toute forme tautomère.

3. Composé selon la revendication 1, ou 2, dans lequel R¹ est un groupe aryle, aryl-alkyle en C₁ à C₆ ou hétaryle facultativement substitué par un ou plusieurs de R⁶.

4. Composé selon l'une quelconque des revendications 1 à 3, dans lequel R² est un groupe alkyle en C₁ à C₆, cycloalkyle en C₃ à C₁₀, cycloalkyl en C₃ à C₁₀-alkyle en C₁ à C₆, trihalogénométhyle, aryl-alkyle en C₁ à C₆ ou hétaryl-alkyle en C₁ à C₆ où les groupes alkyle, cycloalkyle et aryle sont indépendamment facultativement substitués par un ou plusieurs R⁷.

5. Composé selon l'une quelconque des revendications 1 à 4, dans lequel R³ est un groupe alkyle en C₁ à C₆ facultativement substitué par un ou plusieurs de R⁸.

6. Composé selon l'une quelconque des revendications 1 à 5, dans lequel R⁴ est un groupe cycloalkyle en C₆ à C₁₀ ou hetcycloalkyle en C₆ à C₁₀, où les groupes cycloalkyle et hetcycloalkyle sont indépendamment facultativement substitués par un ou plusieurs de R⁸.

7. Composé selon l'une quelconque des revendications 1 à 6, dans lequel R⁶ et R⁷ sont indépendamment un atome d'hydrogène, un groupe hydroxy, oxo, halogéno, cyano, alkyle en C₁ à C₆, alkyloxy en C₁ à C₆, trihalogénométhyle, NR¹⁰R¹¹, aryl-alkyloxy en C₁ à C₆, hétaryl-alkyloxy en C₁ à C₆, alkylcarbonyle en C₁ à C₆, arylcarbonyle, hétarylcarbonyle, aryl-alkylcarbonyle en C₁ à C₆, alkyloxycarbonyle en C₁ à C₆, aryloxycarbonyle ou aryl-alkyloxycarbonyle en C₁ à C₆.

8. Composé selon l'une quelconque des revendications 1 à 7, dans lequel R⁸ et R⁹ sont indépendamment un atome d'hydrogène, un groupe alkyle en C₁ à C₆, hydroxy, oxo, alkyloxy, en C₁ à C₆ ou aryl-alkyloxy en C₁ à C₆.

9. Composé selon l'une quelconque des revendications 1 à 8, dans lequel R¹⁰ et R¹¹ sont indépendamment un atome d'hydrogène ou un groupe alkyle en C₁ à C₆.

10. Composé selon l'une quelconque des revendications 1 à 9 qui est le cyclohexyl-méthyl-amide d'acide 1-(4-chloro-phényl)-5,-propyl-1*H-*pyrazole-4-carboxylique, ou un sel de celui-ci avec un acide ou une base pharmaceutiquement acceptable, ou tout isomère optique ou mélange d'isomères optiques, comprenant un mélange racémique, ou toute forme tautomère.

11. Composé selon l'une quelconque des revendications 1 à 9, choisi dans le groupe constitué par :
le cyclohexyl-méthyl-amide d'acide 1-(4-chloro-phényl)-5-trifiuorométhyl-1H-pyrazole-4-carboxylique ;
la [1-(4-méthoxy-phényl)-5-méthyl-1*H*-pyrazol-4-yl]-(1,3,3-triméthyl-6-aza-bicyclo[3.2.1]oct-6-yl)-méthanone ;
la 1-[1-(4-chloro-phényl)-5-propyl-1*H*-pyrazol-4-yl]-(1,3,3-triméthyl-6-aza-bicyclo[3,2.1]oct-6-yl)-méthanone ;
la [1-(3,5-dichloro-phézayl)-5-propyl-1*H*-pyrazol-4-yl]-(1,3,3-trimétlayl-6-aza-bicydo[3.2.1]oct-6-yl)-méthanone ; ou
sel de celui-ci avec un acide ou une base pharmaceutiquement acceptable, ou tout isomère optique ou mélange d'isomères optiques, comprenant un mélange racémique, ou toute forme tautomère.

12. Composé selon l'une quelconque des revendications 1 à 9, choisi dans le groupe constitué par :
le cyclohexyl-methyl-amide d'acide 1-(phényl)-5-méthyl-1*H*-yrazole-4-carboxylique ;
le cyclohexyl-méthyl-amide d'acide 1-(4-fluoro-phényl)-5-méthyl-1*H-*pyrazole-4-carboxylique ;
le cyclohexyl-méthyl-amide d'acide 1-(4-méthoxy-phênyl)-5-méthyl-1*H-*pyrazole-4-carboxylique ;
le cyclohexyl-méthyl-amide d'acide 1-(4-chloro-phényl)-5-méthyl-1*H-*pyrazole-4-carboxylique ;
le cyclohcxyl-méthyl-amide d'acide 1-(2-méthyl-phényl)-5-méthyl-1*H-*pyrazole-4-carboxylique ;
le cyclohexyl-méthyl-amide d'acide 1-(4-amino-phényl)-5-méthyl-1*H-*pyrazole-4-carboxylique ;
le cyclohexyl-méthyl-amide d'acide 1-(2-pyridyl)-5-méthyl-1*H*-pyrazole-4-carboacylique ;
le cyclohexyl-propyl-amide d'acide 1-(2-pyridyl)-5-propyl-1*H*-pyrazole-4-carboxylique ; ou
sel de celui-ci avec un acide ou une base pharmaceutiquement acceptable, ou tout isomère optique ou mélange d'isomères optiques, comprenant un mélange racémique, ou toute forme tautomère.

13. Combinaison comprenant un composé tel que défini dans l'une quelconque des revendications 1 à 12 et une ou plusieurs substance actives supplémentaires.

14. Combinaison selon la revendication 13, dans laquelle les substances actives supplémentaires sont choisies parmi les agonistes de CART (produit de transcription régulé par la cocaïne et les amphétamines), les antagonistes de NPY (neuropeptide Y), les agonistes de MC4 (mélano-cortine 4), les antagonistes de l'orexine, les agonistes de TNF (facteur nécrosant tumoral), les agonistes de CRF (facteur de libération de corticotropine), les antagonistes de CRF BP (protéine de liaison au facteur de libération de corticotropine), les agonistes d'urocortine, les agonistes β3, les agonistes de MSH (hormone de stimulation des mélanocytes), les antagonistes de MCH (hormone de concentration des mélanocytes), les agonistes de CCK (cholécystokinine), les inhibiteurs de réabsorption de sérotonine, les inhibiteurs de réabsorption de sérotonine et de noradrénaline, des composés mixtes sérotonine et noradrénergiques, les agonistes de 5HT (sérotonine), les agonistes de la bombésine, les antagonistes de la galanine, l'hormone de croissance, les composés de libération d'hormone de croissance, les agonistes de TRH (hormone de libération de thyréotropine), les modulateurs d'UCP 2 ou 3 (protéine de découplage 2 ou 3), les agonistes de la leptine, les agonistes de DA (bromocriptine, doprexine), les inhibiteurs de lipase/amylase, les modulateurs de PPAR (récepteur activé par proliférateur de peroxysome), les modulateurs de RXR (récepteur rétinoïde X), les agonistes de TR β, les inhibiteurs d'AGRP (protéine apparentée à Agouti), les antagonistes d'histamine H3, les antagonistes opioïdes (tels que la naltrexone), l'exendine-4, GLP-1 et le facteur neurotrophique ciliaire.

15. Composition pharmaceutique comprenant, en tant qu'ingrédient actif, au moins un composé selon l'une quelconque des revendications 1 à 12 ou une combinaison selon la revendication 13 ou la revendication 14, conjointement avec un ou plusieurs supports ou excipients pharmaceutiquement acceptables.

16. Composition pharmaceutique selon la revendication 15 qui est destinée à une administration orale, nasale, buccale, transdermique, pulmonaire ou parentérale.

17. Composition pharmaceutique selon la revendication 15 ou 16 sous forme posologique unitaire comprenant 0,05 mg à 2 000 mg/jour, 0,1 mg à 1 000 mg ou 0,5 mg à 500 mg par jour du composé selon l'une quelconque des revendications 1 à 12.

18. Composé tel que défini dans l'une quelconque des revendications 1 à 12, ou combinaison telle que définie dans la revendication 13 ou la revendication 14, ou composition telle que définie dans l'une quelconque des revendications 15 à 17, à utiliser comme médicament.

19. Composé, combinaison ou composition selon la revendication 18 à utiliser dans le traitement, la prévention et/ou la prophylaxie de toute affection, tout trouble et toute pathologie dans lesquels une modulation ou une inhibition de l'activité de 11βHSD1 est bénéfique.

20. Composé, combinaison ou composition selon la revendication 18 à utiliser dans le traitement, la prévention et/ou la prophylaxie de toute affection, tout trouble et toute pathologie influencés par le taux de glucocorticoïde intracellulaire.

21. Composé, combinaison ou composition selon la revendication 18 à utiliser dans le traitement, la prévention et/ou la prophylaxie de toute affection, tout trouble et toute pathologie choisis dans le groupe constitué par le syndrome métabolique, l'insulinorésistance, la dyslipidémie, l'hypertension et l'obésité.

22. Composé, combinaison ou composition selon la revendication 18 à utiliser dans le traitement, la prévention et/ou la prophylaxie du diabète de type 2, de l'intolérance au glucose (IGT), de l'hyperglycémie à jeun (IFG).

23. Composé, combinaison ou composition selon la revendication 18 à utiliser dans le retardement ou la prévention de la progression de l'IGT dans le diabète de type 2.

24. Composé, combinaison ou composition selon la revendication 18 à utiliser dans le retardement ou la prévention de la progression du syndrome métabolique dans le diabète de type 2.

25. Composé, combinaison ou composition selon la revendication 18 à utiliser dans le traitement, la prévention et/ou la prophylaxie d'effets secondaires d'un traitement ou d'une thérapie avec agoniste du récepteur glucocorticoïde.

26. Utilisation d'un composé tel que défini dans l'une quelconque des revendications 1 à 12, ou d'une combinaison telle que définie dans la revendication 13 ou 14 pour la préparation d'un médicament pour le traitement, la prévention et/ou la prophylaxie d'une affection, d'un trouble ou d'une pathologie tel que défini dans l'une quelconque des revendications 19 à 25.

27. Procédé de préparation d'un composé tel que défini dans la revendication 1, ledit procédé comprenant : 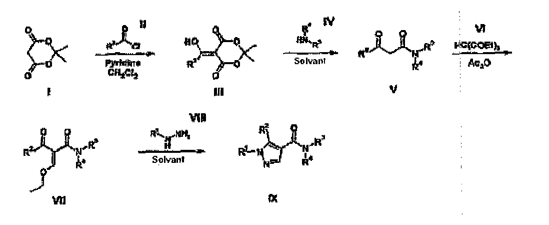 la réaction de 2,2-diméthyl-1,3-dioxane-4,6-dione (acide de Meldrum) (I) avec un chlorure d'acide (II), où R² est défini dans la revendication 1, dans un mélange de solvants de pyridine et de DCM pour donner un acide de Meldrum acyle (III), où R² est tel que défini dans la revendication 1 ; l'aminolyse de l'acide de Meldrum acyle (III) avec une amine substituée (IV), où R³ et R⁴ sont tels que définis dans la revendication 1, dans un solvant tel que le benzène, le toluène, le dioxane à un intervalle de température de 50 °C jusqu'au reflux pour donner un *bêta*-céto amide (V) où R², R³ et R⁴ sont tels que définis dans la revendication 1 ; le traitement du *bêta*-céto amide (V) avec de l'*ortho-*formiate (VI) dans un solvant tel qu'un anhydride d'acide acétique à un intervalle de température de 50 °C jusqu'au reflux pour donner de l'énol éther (VII) où R², R³ et R⁴ sont tels que définis dans la revendication 1 ; la condensation de l'énol éther (VII) où R², R³ et R⁴ sont tels que définis dans la revendication 1 avec un hydrazide (VIII) où R¹ est tel que défini dans la revendication 1 pour donner du pyrazole (IX) où R¹, R², R³ et R⁴ sont tels que définis dans la revendication 1, dans un solvant tel que EtOH, iPrOH, *tert*-BuOH.
